# EUROPEAN PATENT APPLICATION

(11) **EP 4 613 768 A1**
(43) Date of publication of application: **10.09.2025**
(21) Application number: 23885002.8
(22) Date of filing: 01.11.2023
(51) Int. Cl.: C07K 16/28, C07K 19/00, C12N 15/13, C12N 15/63, C12N 5/10, A61K 39/395, A61P 3/04, A61P 3/10

(54) **GIPR BINDING PROTEIN AND USE THEREOF**

(30) Priority: 02.11.2022 CN 202211365864
(71) Applicant: Suzhou Alphamab Co., Ltd, Jiangsu 215125 (CN)
(72) Inventor: XU, Ting, Suzhou, Jiangsu 215125 (CN); JIN, Yuhao, Suzhou, Jiangsu 215125 (CN); ZHU, Xuguo, Suzhou, Jiangsu 215125 (CN); PANG, Minjie, Suzhou, Jiangsu 215125 (CN); YUN, Lihong, Suzhou, Jiangsu 215125 (CN)
(74) Representative: Maiwald GmbH
(86) International application number: PCT/CN2023/129127
(87) International publication number: WO 2024/094076

(57) **Abstract**

Provided are a GIPR binding protein and a use thereof. Specifically provided are a single-domain antibody specifically binding to a glucose-dependent insulinotropic polypeptide receptor, a derived protein thereof, and a pharmaceutical use.

## Description

### TECHNICAL FIELD

The present disclosure relates to the field of biopharmaceutics and discloses a single-domain antibody against glucose-dependent insulinotropic polypeptide receptor (GIPR) and derivative proteins thereof. Specifically, the present disclosure discloses a binding protein against glucose-dependent insulinotropic polypeptide receptor and use thereof.

### BACKGROUND

Glucose-dependent insulinotropic polypeptide (GIP) is an enterogenic incretin hormone that can enhance glucose-stimulated insulin secretion. GIP is secreted by K cells located in the proximal small intestine, and its secretion in humans is effectively stimulated by dietary carbohydrates and fats. Research shows that rodents fed on high-fat diets exhibit increased GIP secretion from K cells. Similarly, humans' acute high intakes of fats increase GIP concentrations by 42%, followed by significant weight gain, indicating a positive correlation between high-fat diet exposure and GIP concentrations. Thus, high-fat diets and elevated systemic GIP concentrations may be the basis for the difference in obesity among human subjects.

Recent human genetic studies have revealed associations between glucose-dependent insulinotropic polypeptide receptor (GIPR) and body mass index (BMI). In addition, GIPR knockout mice displaying resistance to high-fat diet-induced obesity, coupled with the established roles of GIPR in pancreatic cells and adipocytes, indicates that the development of GIPR antagonists for treating obesity appears to be a viable strategy. However, there is still a lack of GIPR antagonists with clearly demonstrated therapeutic effects in the prior art.

### SUMMARY

In the present disclosure, an anti-GIPR single-domain antibody (VHH) and derivative proteins thereof are obtained through screening utilizing phage display technology. The anti-GIPR single-domain antibody (VHH) and the derivative proteins thereof of the present disclosure have significant effects of high specificity and high affinity.

In a first aspect, the present disclosure provides a glucose-dependent insulinotropic polypeptide receptor (GIPR)-binding protein comprising at least one immunoglobulin single variable domain, wherein the at least one immunoglobulin single variable domain comprises a CDR1, a CDR2, and a CDR3 from a VHH set forth in SEQ ID NO: 5 and/or SEQ ID NO: 6.

In some embodiments, the CDR1, CDR2, and CDR3 are defined according to the following definition system: Kabat, AbM, Chothia, or IMGT.

In some embodiments, the immunoglobulin single variable domain is from the family Camelidae, humanized, or chimeric.

In some embodiments, the CDR1, CDR2, and CDR3 from the VHH set forth in SEQ ID NO: 5 are selected from any one of the following groups: SEQ ID NOs: 74-76, SEQ ID NOs: 77-79, SEQ ID NOs: 80-82, and SEQ ID NOs: 83-85. In some embodiments, the CDR1, CDR2, and CDR3 from the VHH set forth in SEQ ID NO: 5 are selected from any one of the following groups: SEQ ID NOs: 74-76, SEQ ID NOs: 77-79, SEQ ID NOs: 80-82, and SEQ ID NOs: 83-85. In some embodiments, the at least one immunoglobulin single variable domain comprises one or more of the amino acid sequences set forth in SEQ ID NO: 5 and SEQ ID NOs: 8-14. In some embodiments, the at least one immunoglobulin single variable domain comprises the amino acid sequence set forth in one of SEQ ID NO: 5 and SEQ ID NOs: 8-14.

In some embodiments, the CDR1, CDR2, and CDR3 from the VHH set forth in SEQ ID NO: 6 are selected from any one of the following groups: SEQ ID NOs: 86-88, SEQ ID NOs: 89-91, SEQ ID NOs: 92-94, and SEQ ID NOs: 95-97. In some embodiments, the at least one immunoglobulin single variable domain comprises one or more of the amino acid sequences set forth in SEQ ID NO: 6 and SEQ ID NOs: 15-25. In some embodiments, the at least one immunoglobulin single variable domain comprises the amino acid sequence set forth in one of SEQ ID NO: 6 and SEQ ID NOs: 15-25.

In some embodiments, the at least one immunoglobulin single variable domain comprises one or more of the amino acid sequences set forth in SEQ ID NO: 5 and SEQ ID NOs: 8-14, and one or more of the amino acid sequences set forth in SEQ ID NO: 6 and SEQ ID NOs: 15-25. In some embodiments, the at least one immunoglobulin single variable domain comprises the amino acid sequence set forth in one of SEQ ID NO: 5 and SEQ ID NOs: 8-14 and the amino acid sequence set forth in one of SEQ ID NO: 6 and SEQ ID NOs: 15-25.

In some embodiments, the GIPR-binding protein comprises one said immunoglobulin single variable domain. In some embodiments, the GIPR-binding protein comprises multiple, for example, 2, 3, 4, or 5, said immunoglobulin single variable domains.

In some embodiments, the GIPR-binding protein further comprises an immunoglobulin Fc region, preferably a human immunoglobulin Fc region, and more preferably a human IgG1, IgG2, IgG3, or IgG4 Fc region. In some embodiments, the immunoglobulin Fc region comprises the amino acid sequence set forth in SEQ ID NO: 110 or SEQ ID NO: 113. In some embodiments, the amino acid sequence of the immunoglobulin Fc region is set forth in SEQ ID NO: 110 or SEQ ID NO: 113.

In some embodiments, the immunoglobulin Fc region is linked directly or linked indirectly by a linker to the at least one immunoglobulin single variable domain.

In some embodiments, the GIPR-binding protein has at least one of the following characteristics:
(a) having specific binding to human GIPR;
(b) binding to human GIPR with a KD value of less than 1 × 10⁻⁷ M, preferably less than 1 × 10⁻⁸ M;
(c) blocking the interaction of GIP with GIPR; and
(d) being capable of reducing the body weight and/or blood glucose of a subject.

In a second aspect, the present disclosure provides a fusion protein comprising the GIPR-binding protein according to the first aspect of the present disclosure.

In a third aspect, the present disclosure provides a multispecific antibody comprising the GIPR-binding protein according to the first aspect of the present disclosure and/or the fusion protein according to the second aspect of the present disclosure, and one or more additional antigen-binding regions that, compared to the GIPR-binding protein and/or the fusion protein, bind to a different antigen or a different epitope of the same antigen.

In a fourth aspect, the present disclosure provides a conjugate comprising the GIPR-binding protein according to the first aspect of the present disclosure and/or the fusion protein according to the second aspect of the present disclosure and/or the multispecific antibody according to the third aspect of the present disclosure, wherein the conjugate further comprises a molecule conjugated to the GIPR-binding protein and/or the fusion protein and/or the multispecific antibody.

In a fifth aspect, the present disclosure provides a nucleic acid molecule encoding the GIPR-binding protein according to the first aspect of the present disclosure and/or the fusion protein according to the second aspect of the present disclosure and/or the multispecific antibody according to the third aspect of the present disclosure.

In a sixth aspect, the present disclosure provides an expression vector comprising the nucleic acid molecule according to the fifth aspect of the present disclosure which is operably linked to an expression control element.

In a seventh aspect, the present disclosure provides a recombinant cell comprising the nucleic acid molecule according to the fifth aspect of the present disclosure and/or transformed with the expression vector according to the sixth aspect of the present disclosure and capable of expressing the GIPR-binding protein and/or the fusion protein and/or the multispecific antibody.

In an eighth aspect, the present disclosure provides a pharmaceutical composition comprising the GIPR-binding protein according to the first aspect of the present disclosure and/or the fusion protein according to the second aspect of the present disclosure and/or the multispecific antibody according to the third aspect of the present disclosure and/or the conjugate according to the fourth aspect of the present disclosure and/or the nucleic acid molecule according to the fifth aspect of the present disclosure and/or the expression vector according to the sixth aspect of the present disclosure and/or the recombinant cell according to the seventh aspect of the present disclosure and a pharmaceutically acceptable carrier.

In a ninth aspect, the present disclosure provides a kit comprising the GIPR-binding protein according to the first aspect of the present disclosure, the fusion protein according to the second aspect of the present disclosure, the multispecific antibody according to the third aspect of the present disclosure, the conjugate according to the fourth aspect of the present disclosure, and/or the pharmaceutical composition according to the eighth aspect of the present disclosure.

In a tenth aspect, the present disclosure provides a method for determining the presence and/or content of a GIPR protein, comprising providing the GIPR-binding protein according to the first aspect of the present disclosure, the fusion protein according to the second aspect of the present disclosure, the multispecific antibody according to the third aspect of the present disclosure, and/or the conjugate according to the fourth aspect of the present disclosure.

In an eleventh aspect, the present disclosure provides a method for inhibiting binding of a GIPR protein to a ligand thereof, comprising providing the GIPR-binding protein according to the first aspect of the present disclosure, the fusion protein according to the second aspect of the present disclosure, the multispecific antibody according to the third aspect of the present disclosure, and/or the conjugate according to the fourth aspect of the present disclosure. In a preferred embodiment, the ligand is GIP.

In a twelfth aspect, the present disclosure provides a method for treating and/or preventing a metabolic disease and/or symptom, comprising administering to a subject in need thereof an effective amount of the GIPR-binding protein according to the first aspect of the present disclosure, the fusion protein according to the second aspect of the present disclosure, the multispecific antibody according to the third aspect of the present disclosure, the conjugate according to the fourth aspect of the present disclosure, and/or the pharmaceutical composition according to the eighth aspect of the present disclosure. In some embodiments, the metabolic disease and/or symptom is obesity, overweight, and/or diabetes.

In a thirteenth aspect, the present disclosure provides use of the GIPR-binding protein according to the first aspect of the present disclosure, the fusion protein according to the second aspect of the present disclosure, the multispecific antibody according to the third aspect of the present disclosure, the conjugate according to the fourth aspect of the present disclosure, and/or the pharmaceutical composition according to the eighth aspect of the present disclosure in the manufacture of a medicament for treating and/or preventing a metabolic disease and/or symptom. In some embodiments, the metabolic disease and/or symptom is obesity, overweight, and/or diabetes.

In a fourteenth aspect, the present disclosure provides the GIPR-binding protein according to the first aspect of the present disclosure, the fusion protein according to the second aspect of the present disclosure, the multispecific antibody according to the third aspect of the present disclosure, the conjugate according to the fourth aspect of the present disclosure, and/or the pharmaceutical composition according to the eighth aspect of the present disclosure for use in the treatment and/or prevention a metabolic disease and/or symptom. In some embodiments, the metabolic disease and/or symptom is obesity, overweight, and/or diabetes.

Other aspects and advantages of the present disclosure will be readily apparent to those skilled in the art from the following detailed description. Only exemplary embodiments of the present disclosure are shown and described in the following detailed description. As those skilled in the art will recognize, the content of the present disclosure enables those skilled in the art to make changes to the specific embodiments disclosed without departing from the spirit and scope of the invention to which the present disclosure relates. Accordingly, the descriptions in the drawings and specification of the present disclosure are only illustrative rather than limiting.

### BRIEF DESCRIPTION OF THE DRAWINGS

Specific features of the invention to which the present disclosure relates are set forth in appended claims. Features and advantages of the invention to which the present disclosure relates will be better understood with reference to the exemplary embodiments and drawings described in detail below. The drawings are briefly described below:
FIG. 1 shows the blocking activity of a GIPR single-domain antibody-Fc fusion protein against GIPR, wherein △ represents iGI-72-Ld-Fc, and ∘ represents AMG-GIPR-mab2 as a positive control.
FIG. 2 shows the effect of multiple administrations of a GIPR single-domain antibody-Fc fusion protein on the body weight of DIO mice.
FIG. 3 shows the effect of multiple administrations of a GIPR single-domain antibody-Fc fusion protein on fasting blood glucose levels in DIO mice.
FIG. 4 shows the effect of multiple administrations of a GIPR single-domain antibody-Fc fusion protein on insulin secretion in DIO mice.
FIG. 5 shows the effect of multiple administrations of a GIPR single-domain antibody-Fc fusion protein on the insulin resistance index in DIO mice.
FIG. 6 shows the neutralizing activity of humanized GIPR single-domain antibody-Fc fusion proteins.
FIG. 7 shows the effect of a humanized GIPR single-domain antibody-Fc fusion protein on blood glucose levels in DA-GIP-stimulated C57BL/6 mice.
FIG. 8 shows the effect of a humanized GIPR single-domain antibody-Fc fusion protein on insulin secretion in DA-GIP-stimulated C57BL/6 mice.
FIG. 9 shows the effect of multiple administrations of a humanized GIPR single-domain antibody-Fc fusion protein on the body weight of ob mice.
FIG. 10 shows the abilities of a humanized GIPR single-domain antibody-Fc fusion protein and maridebart to bind to GIPR.
FIG. 11 shows the abilities of a humanized GIPR single-domain antibody-Fc fusion protein and maridebart to neutralize GIPR.

### DETAILED DESCRIPTION

The embodiments of the invention of the present disclosure are described below with reference to specific examples, and other advantages and effects of the invention of the present disclosure will be readily apparent to those skilled in the art from the disclosure of the present specification.

### Definitions of Terms

Unless otherwise indicated or defined, all terms used herein have the ordinary meaning in the art that would be understood by those skilled in the art. Reference is made, for example, to standard manuals, such as Sambrook et al., "Molecular Cloning: A Laboratory Manual" (2nd edition), Vol. 1-3, Cold Spring Harbor Laboratory Press (1989); Lewin, "Genes IV", Oxford University Press, New York, (1990); and Roitt et al., "Immunology" (2nd edition), Gower Medical Publishing, London, New York (1989), and general prior art cited herein; moreover, unless otherwise indicated, all methods, steps, techniques, and operations not specifically recited may be, and have been, performed in a manner known per se to those skilled in the art. Reference is also made, for example, to standard manuals, the general prior art described above, and other references cited therein.

Unless otherwise indicated, the terms "antibody" and "immunoglobulin" are used interchangeably herein and, whether referring to a heavy-chain antibody or to a conventional 4-chain antibody, are used as a general term to include full-length antibodies, individual chains thereof, and all portions, domains, or fragments thereof (including but not limited to antigen-binding domains or fragments, such as VHH domains or VH/VL domains, respectively). In addition, the term "sequence" as used herein (e.g., in the term "immunoglobulin sequence", "antibody sequence", "single variable domain sequence", "VHH sequence", or "protein sequence") generally should be understood to include not only related amino acid sequences but also nucleic acid or nucleotide sequences encoding the sequences, unless further limited interpretation is required herein.

As used herein, the term "domain" (of a polypeptide or protein) refers to a folded protein structure that is capable of maintaining its tertiary structure independently of the rest of the protein. In general, a domain is responsible for a single functional property of a protein and, in many cases, may be added, removed, or transferred to other proteins without compromising the functionality of the rest of the protein and/or the domain.

The term "immunoglobulin domain" as used herein refers to a globular region of an antibody chain (e.g., a chain of a conventional 4-chain antibody or a chain of a heavy-chain antibody) or a polypeptide essentially consisting of such globular regions. An immunoglobulin domain is characterized in that it maintains the immunoglobulin folding characteristics of an antibody molecule. The term "immunoglobulin variable domain" as used herein refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are spaced apart by three "complementarity-determining regions" or "CDRs" referred to in the art and hereinafter as "complementarity-determining region 1" or "CDR1", "complementarity-determining region 2" or "CDR2", and "complementarity-determining region 3" or "CDR3". Thus, the general structure or sequence of an immunoglobulin variable domain can be represented as follows: FR1-CDR1-FR2-CDR2-FR3-CDR3-FR4. An immunoglobulin variable domain gives an antibody specificity for an antigen due to the antigen-binding site it has.

The term "immunoglobulin single variable domain" as used herein refers to an immunoglobulin variable domain that is capable of specifically binding to an epitope of an antigen without pairing with other immunoglobulin variable domains. An example of the immunoglobulin single variable domain of the present disclosure is "domain antibody", such as immunoglobulin single variable domains VH and VL (VH domain and VL domain). Another example of the immunoglobulin single variable domain is a "VHH domain" (or "VHH" for short) of the family Camelidae as defined below. The "VHH domain", also known as heavy chain single-domain antibody, VHH, VHH antibody fragment, and VHH antibody, is the variable domain of antigen-binding immunoglobulin known as "heavy-chain antibody" (i.e., "antibody devoid of light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)). The term "VHH domain" is used to distinguish the variable domain from the heavy chain variable domains (referred to herein as "VH domain") present in conventional 4-chain antibodies and the light chain variable domains (referred to herein as "VL domain") present in conventional 4-chain antibodies. A VHH domain specifically binds to an epitope without the need for an additional antigen-binding domain (as opposed to the VH or VL domain in a conventional 4-chain antibody, in which case the epitope is recognized by the VL domain together with the VH domain). A VHH domain is a small, stable, and efficient antigen recognition unit formed by a single immunoglobulin domain.

In the context of the present disclosure, the terms "heavy-chain single-domain antibody", "VHH domain", "VHH", "VHH antibody fragment", and "VHH antibody" are used interchangeably.

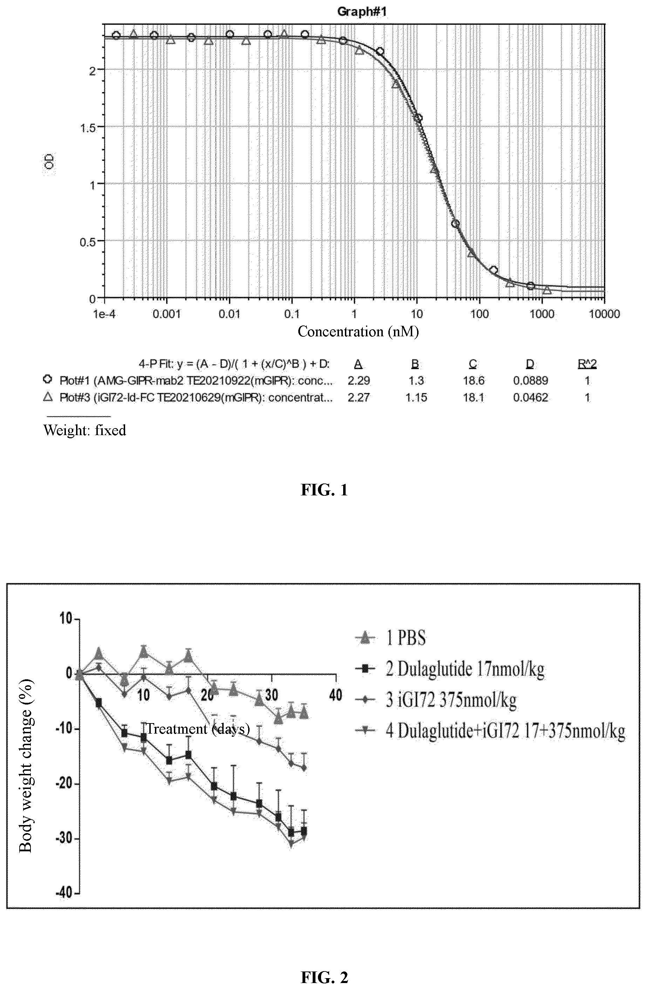

For example, as shown in FIG. 2 in Riechmann and Muyldermans, J. Immunol. Methods 231, 25-38 (1999), the amino acid residues used in VHH domains of the family Camelidae can be numbered according to the general numbering method of VH domains given by Kabat et al. (Kabat et al., Sequences of Proteins of Immunological Interest, 5th Ed. Public Health Service, National Institutes of Health, Bethesda, Md. (1991)).

Alternative methods for numbering the amino acid residues of VH domains are known in the art and may also be similarly applied to VHH domains. For example, Chothia CDRs refer to the positions of structural loops (Chothia and Lesk, J. Mol. Biol. 196:901-917 (1987)). AbM CDRs represent a compromise of Kabat hypervariable regions and Chothia structure loops and are used in Oxford Molecular's AbM antibody modeling software. "Contact" CDRs are based on the analysis of available complex crystal structures. The residues of CDRs from the methods are described in Table 1 below:

**Table 1. The residues of CDRs according to various numbering systems**

| Loop | Kabat | AbM | Chothia | Contact |
|---|---|---|---|---|
| LCDR1 | L24-L34 | L24-L34 | L26-L32 | L30-L36 |
| LCDR2 | L50-L56 | L50-L56 | L50-L52 | L46-L55 |
| LCDR3 | L89-L97 | L89-L97 | L91-L96 | L89-L96 |
| HCDR1 (Kabat numbering) | H31-H35B | H26-H35B | H26-H32 | H30-H35B |
| HCDR1 (Chothia numbering) | H31-H35 | H26-H35 | H26-H32 | H30-H35 |
| HCDR2 | H50-H65 | H50-H58 | H53-H55 | H47-H58 |
| HCDR3 | H95-H102 | H95-H102 | H96-H101 | H93-H101 |

The CDRs of an antibody may also be IMGT-CDRs. This is a CDR definition method based on the IMGT antibody numbering, which is obtained by integrating structural information of over 5000 sequences. In the VH CDR numbering of IMGT, CDR1: 27-38; CDR2: 56-65; CDR3:105-117.

However, it should be noted that, as is well known in the art for VH domains and for VHH domains, the total number of amino acid residues in each CDR may vary and may not correspond to the total number of amino acid residues indicated by the Kabat numbering (that is, one or more positions according to the Kabat numbering may not be occupied in the actual sequence, or the actual sequence may contain more amino acid residues than the Kabat numbering allows). This means that, generally, the numbering according to Kabat may or may not correspond to the actual numbering of the amino acid residues in the actual sequence.

For example, CDRs may include "extended CDRs", such as 24-36 or 24-34 (LCDR1), 46-56 or 50-56 (LCDR2), and 89-97 or 89-96 (LCDR3) in the VL; and 26-35 (HCDR1), 50-65 or 49-65 (HCDR2), and 93-102, 94-102, or 95-102 (HCDR3) in the VH.

The total number of amino acid residues in a VHH domain generally will be in the range of 110 to 120, often between 112 and 115. However, it should be noted that smaller and longer sequences may also be suitable for the purposes described herein.

Other structural characteristics and functional properties of VHH domains and polypeptides comprising the same can be summarized as follows:
VHH domains (which have been "designed" by nature to functionally bind to an antigen in the absence of and without interaction with a light chain variable domain) can be used as a single and relatively small functional antigen-binding structural unit, domain, or polypeptide. The characteristics distinguish VHH domains from the VH and VL domains of conventional 4-chain antibodies. These VH and VL domains by themselves are generally not suited for practical application as single antigen-binding proteins or immunoglobulin single variable domains, but need to be combined in some form or another to provide a functional antigen-binding unit (e.g., in the form of a conventional antibody fragment such as a Fab fragment, or in the form of an scFv consisting of a VH domain covalently linked to a VL domain).

Due to these unique properties, the use of VHH domains-either alone or as part of a larger polypeptide-offers many significant advantages over the use of conventional VH and VL domains, scFvs, or conventional antibody fragments (such as Fab- or F(ab')2-fragments): only a single domain is required to bind to an antigen with high affinity and high selectivity, so that there is no need to have two separate domains present, nor to assure that the two domains are present in the suitable spacial conformation and configuration (e.g., the use of specially designed linkers is generally required for scFvs); VHH domains can be expressed from a single gene and require no post-translational folding or modifications; VHH domains can be easily engineered into multivalent and multispecific formats (formatting); VHH domains are highly soluble and do not have a tendency to aggregate; VHH domains are highly stable to heat, pH, proteases, and other denaturing agents or conditions and thus can be prepared, stored, or transported without the use of refrigeration equipment, thereby achieving cost, time, and environmental savings; VHH domains are easy to prepare and are relatively cheap, even on a scale required for production; VHH domains are relatively small (approximately 15 kDa or 10 times smaller than a conventional IgG) compared to conventional 4-chain antibodies and antigen-binding fragments thereof and thus show higher tissue permeability and can be administered at higher doses than conventional 4-chain antibodies and antigen-binding fragments thereof; VHH domains can show so-called cavity-binding properties (especially due to their extended CDR3 loop, compared to conventional VH domains) and can therefore access targets and epitopes not accessible to conventional 4-chain antibodies and antigen-binding fragments thereof.

Methods of obtaining VHHs binding to a specific antigen or epitope have been disclosed earlier in R. van der Linden et al., Journal of Immunological Methods, 240(2000) 185-195; Li et al., J Biol Chem., 287(2012) 13713-13721; Deffar et al., African Journal of Biotechnology Vol. 8(12), pp. 2645-2652, 17 June, 2009, and WO94/04678.

VHH domains derived from the family Camelidae can be "humanized" (also referred to herein as "sequence optimization"; in addition to humanization, "sequence optimization" may encompass other modifications to the sequence through one or more mutations providing improved properties of the VHH, such as removal of potential post-translational modification sites) by replacing one or more amino acid residues in the amino acid sequence of the original VHH sequence with one or more of the amino acid residues that exist at the corresponding position(s) in a VH domain of a conventional human 4-chain antibody. The humanized VHH domain may comprise one or more fully human framework region sequences. Humanization can be accomplished using methods for protein surface amino acid humanization (resurfacing) and/or humanized CDR grafting to a universal framework, for example, as exemplified in the examples.

In general, the term "specificity" refers to the number of different types of antigens or epitopes to which a particular antigen-binding molecule or antigen-binding protein (e.g., the immunoglobulin single variable domain, heavy-chain single-domain antibody, or GIPR-binding protein of the present disclosure) can bind. The specificity of an antigen-binding protein can be determined based on its affinity and/or avidity. Affinity, represented by a dissociation equilibrium constant (KD) of an antigen to an antigen-binding protein, is a measure of the binding strength between an epitope and an antigen-binding site on the antigen-binding protein: a smaller KD value indicates a stronger binding strength between the epitope and the antigen-binding protein (or, the affinity can also be expressed as an association constant (KA), which is 1/KD). As will be appreciated by those skilled in the art, affinity can be determined in a known manner depending on the particular antigen of interest. Avidity is a measure of the binding strength between an antigen-binding protein (e.g., an immunoglobulin, an antibody, an immunoglobulin single variable domain, or a polypeptide comprising the same) and a related antigen. Avidity is related to both the affinity of antigen-binding sites on the antigen-binding protein for the related antigen and the number of related binding sites present on the antigen-binding protein.

As used herein, the term "glucose-dependent insulinotropic polypeptide receptor", abbreviated as GIPR, encompasses the GIPR of any species. Preferably, GIPR is human GIPR or mouse GIPR.

As used herein, the term "glucose-dependent insulinotropic polypeptide receptor (GIPR)-binding protein" means any protein capable of specifically binding to glucose-dependent insulinotropic polypeptide receptor (GIPR). GIPR-binding proteins may include heavy-chain single-domain antibodies against GIPR as defined herein. GIPR-binding proteins also encompass immunoglobulin superfamily antibodies (IgSF) or CDR-grafted molecules.

The "GIPR-binding protein" of the present disclosure may comprise at least one immunoglobulin single variable domain, such as VHH, that binds to GIPR. In some embodiments, the "GIPR-binding protein" of the present disclosure may comprise 2, 3, 4, or more immunoglobulin single variable domains, such as VHHs, that bind to GIPR. The GIPR-binding protein of the present disclosure may comprise, in addition to the immunoglobulin single variable domain(s) that bind(s) to GIPR, a linker and/or a moiety with an effector function, such as a half-life extending moiety (e.g., an immunoglobulin single variable domain that binds to serum albumin), and/or a fusion partner (e.g., serum albumin) and/or a conjugated polymer (e.g., PEG) and/or an Fc region. In some embodiments, the "GIPR-binding protein" of the present disclosure also encompasses bispecific antibodies that comprise an antigen-binding domain or immunoglobulin single variable domain that binds to a different antigen or a different region (e.g., a different epitope) of the same antigen.

Generally, the GIPR-binding protein of the present disclosure may bind to an antigen of interest (i.e., GIPR) with a dissociation constant (KD) of preferably 10⁻⁷ to 10⁻¹⁰ mol/L (M), more preferably 10⁻⁸ to 10⁻¹⁰ mol/L, and even more preferably 10⁻⁹ to 10⁻¹⁰ or less, and/or with an association constant (KA) of at least 10⁷ M⁻¹, preferably at least 10⁸ M⁻¹, more preferably at least 10⁹ M⁻¹, and more preferably at least 10¹⁰ M⁻¹, as measured by a Biacore, KinExA, or Fortibio assay. Any KD value greater than 10⁻⁴ M is generally considered to indicate a non-specific binding. The specific binding of an antigen-binding protein to an antigen or epitope can be determined in any known suitable way, including, for example, the surface plasmon resonance (SPR) assay, Scatchard assay, and/or competitive binding assay (e.g., radioimmunoassay (RIA), enzyme immunoassay (EIA), and sandwich competitive assay) described herein.

Amino acid residues will be represented according to the standard three-letter or one-letter amino acid code as well known and agreed upon in the art. When two amino acid sequences are compared, the term "amino acid difference" refers to the insertion, deletion, or substitution of a specified number of amino acid residues at a certain position of the reference sequence as compared with another sequence. In the case of a substitution, the substitution will be preferably a conservative amino acid substitution which means that an amino acid residue is replaced with another amino acid residue that is chemically similar in structure and that has little or no effect on the function, activity or other biological properties of the polypeptide. The conservative amino acid substitution is well known in the art; for example, the conservative amino acid substitution is preferably a substitution of one amino acid within the following groups (i)-(v) with another amino acid residue within the same group: (i) smaller aliphatic nonpolar or weakly polar residues: Ala, Ser, Thr, Pro, and Gly; (ii) polar negatively-charged residues and their (uncharged) amides: Asp, Asn, Glu, and Gln; (iii) polar positively-charged residues: His, Arg, and Lys; (iv) larger aliphatic nonpolar residues: Met, Leu, Ile, Val, and Cys; and (v) aromatic residues: Phe, Tyr, and Trp. Particularly preferred conservative amino acid substitutions are as follows: Ala is substituted with Gly or Ser; Arg is substituted with Lys; Asn is substituted with Gln or His; Asp is substituted with Glu; Cys is substituted with Ser; Gln is substituted with Asn; Glu is substituted with Asp; Gly is substituted with Ala or Pro; His is substituted with Asn or Gln; Ile is substituted with Leu or Val; Leu is substituted with Ile or Val; Lys is substituted with Arg, Gln, or Glu; Met is substituted with Leu, Tyr, or Ile; Phe is substituted with Met, Leu, or Tyr; Ser is substituted with Thr; Thr is substituted with Ser; Trp is substituted with Tyr; Tyr is substituted with Trp or Phe; Val is substituted with Ile or Leu.

The "sequence identity" between two polypeptide sequences indicates the percentage of identical amino acids between the sequences. "Sequence similarity" indicates the percentage of amino acids that are identical or represent conservative amino acid substitutions. Methods of evaluating the degree of sequence identity between amino acids or nucleotides are known to those skilled in the art. For example, amino acid sequence identity is generally measured using sequence analysis software. For example, the BLAST program from the NCBI database can be used to determine the identity. For the determination of sequence identity, see, for example, Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987 and Sequence Analysis Primer, Gribskov, M. and Devereux, J., eds., M Stockton Press, New York, 1991.

In the present disclosure, the term "isolated" generally refers to a biological material (such as a virus, a nucleic acid, or a protein) that is substantially free from components that normally accompany or interact with it in its naturally occurring environment. The isolated biological material optionally comprises an additional material that the biological material is not found to have in its natural environment (such as a nucleic acid or a protein). In the present disclosure, "isolated", when referring to a protein, generally means that the molecule is separate and discrete from the whole organism in which the molecule is found to naturally occur, or other biological macromolecules of the same type are substantially absent. When referring to a nucleic acid molecule, it is completely or partially separate from sequences that naturally bind to it, or the nucleic acid has a heterologous sequence that binds to it, or the nucleic acid is separate from the chromosome.

Compared with the natural biological source of a polypeptide or nucleic acid molecule and/or the reaction medium or culture medium used for obtaining the polypeptide or nucleic acid molecule, when the polypeptide or nucleic acid molecule has been isolated from at least one other usually associated component (such as another protein/polypeptide, another nucleic acid, another biological component or macromolecule or at least one pollutant, impurity or trace component) in the source or medium (culture medium), the polypeptide or nucleic acid molecule is regarded as "isolated". In particular, a polypeptide or nucleic acid molecule is considered "isolated" when it has been purified at least 2-fold, in particular at least 10-fold, more in particular at least 100-fold and up to 1000-fold or more than 1000-fold. The "isolated" polypeptide or nucleic acid molecule is preferably substantially homogeneous, as determined by suitable techniques (e.g., suitable chromatographic techniques, such as polyacrylamide gel electrophoresis).

"Effective amount" means that the amount of the GIPR-binding protein or pharmaceutical composition of the present disclosure can result in a reduction in the severity of the disease symptom, an increase in the frequency and duration of asymptomatic periods of the disease, or the prevention of damages or disability resulting from the affliction of the disease.

As used herein, "metabolic disease" refers to a disorder that affects the production of energy by human (or animal) cells, also known as a metabolic disorder. Most metabolic diseases are inherited diseases, and some arise from diet, toxins, and infections. Common metabolic diseases fall into three main categories: disorders that affect carbohydrate metabolism, disorders that affect fat metabolism, and disorders that affect mitochondria within cells.

The term "subject" as used herein refers to a mammal, particularly a primate, and particularly a human.

In the present disclosure, the term "antigen-binding protein" generally refers to a protein comprising a moiety that binds to an antigen, and optionally a scaffold or framework moiety that allows the antigen-binding moiety to adopt a conformation that facilitates binding of the antigen-binding protein to the antigen. An antigen-binding protein may typically comprise an antibody light chain variable region (VL) or an antibody heavy chain variable region (VH), or both, and functional fragments thereof. In the present disclosure, the term "antigen-binding protein" also encompasses single-domain antibodies as well as proteins comprising immunoglobulin single variable domains. The variable regions of the heavy and light chains comprise binding domains that interact with antigens. Examples of antigen-binding proteins include, but are not limited to, antibodies, antigen-binding fragments, single-domain antibodies, immunoconjugates, multispecific antibodies (e.g., bispecific antibodies), antibody fragments, antibody derivatives, antibody analogs, or fusion proteins, etc., as long as they exhibit the desired antigen-binding activity.

In the present disclosure, the terms "polypeptide" or "protein" are used interchangeably and generally refer to a polymer of amino acid residues. The term also applies to amino acid polymers in which one or more amino acid residues are analogs or mimetics of corresponding naturally occurring amino acids, as well as to naturally occurring amino acid polymers. The term may also include amino acid polymers that are modified, for example, by addition of sugar residues to form glycoproteins or by phosphorylation. Polypeptides and proteins can be produced by naturally occurring and non-recombinant cells or by genetically engineered or recombinant cells, and may comprise molecules having the amino acid sequences of natural proteins, or molecules having one or more amino acid deletions, additions, and/or substitutions of natural sequences. The terms "polypeptide" and "protein" particularly include sequences in which one or more amino acids of the antigen-binding protein described in the present disclosure are deleted, added, and/or substituted.

In the present disclosure, the term "conjugate" generally refers to a substance formed by linking an antigen-binding protein to other active agents, which may be small-molecule active agents, such as therapeutic agents, imaging probes, or spectroscopic probes.

In the present disclosure, the term "nucleic acid" molecule generally refers to nucleotides, deoxyribonucleotides, or ribonucleotides or analogs thereof in an isolated form and of any length that are isolated from its natural environment or artificially synthesized.

In the present disclosure, the term "vector" generally refers to a nucleic acid molecule that is capable of self-replicating in a suitable host and transfers an inserted nucleic acid molecule into a host cell and/or between host cells. The vector may include a vector for primarily inserting DNA or RNA into a cell, a vector for primarily replicating DNA or RNA, and a vector for primarily expressing transcription and/or translation of DNA or RNA. The vector also includes vectors having multiple functions as described above. The vector may be a polynucleotide capable of being transcribed and translated into a polypeptide when introduced into a suitable host cell. Generally, the vector can produce the desired expression product by culturing an appropriate host cell comprising the vector.

In the present disclosure, the term "cell" generally refers to an individual cell, cell line, or cell culture that may contain or already contains a plasmid or vector comprising the nucleic acid molecule described in the present disclosure, or that is capable of expressing the antigen-binding protein described in the present disclosure. The cell may comprise the progeny of a single host cell. Due to natural, accidental, or deliberate mutations, progeny cells may not necessarily be identical in morphology or genome to the original parent cell, but are capable of expressing the antibody or the antigen-binding fragment thereof described in the present disclosure. The cell can be obtained by transfecting a cell with the vector described in the present disclosure *in vitro.* The cell may be a prokaryotic cell (e.g., E. coli) or a eukaryotic cell (e.g., a yeast cell, a COS cell, a Chinese hamster ovary (CHO) cell, a HeLa cell, an HEK293 cell, a COS-1 cell, an NS0 cell, or a myeloma cell). In certain cases, the cell may be a mammalian cell. For example, the mammalian cell may be a CHO-K1 cell.

In the present disclosure, the term "pharmaceutical composition" generally refers to a formulation that is present in a form that allows the biological activity of the active ingredient to be effective, and that does not comprise additional ingredients that have unacceptable toxicity to the subject to which the composition is to be administered.

In the present disclosure, the term "treating" or "treatment" generally refers to a clinical intervention that is intended to alter the natural course of a disease in the individual being treated and can be performed for prevention and treatment or during the course of clinical pathology. Desirable therapeutic effects include, but are not limited to, preventing occurrence or recurrence of the disease, palliating symptoms, diminishing any direct or indirect pathological consequences of the disease, preventing metastasis, reducing the rate of disease progression, ameliorating or alleviating the disease state, and remission or improving prognosis. In some cases, antigen-binding proteins (e.g., antibodies against the particular antigen of the present disclosure) can be used to delay disease progression or slow disease progression.

In the present disclosure, the term "administering" or "administration" generally refers to a method of giving a subject (e.g., a patient) a certain dose of a compound or pharmaceutical composition. Administration may be performed by any suitable means, including parenteral, intrapulmonary, and intranasal, as well as (if topical treatment is desired) intralesional administration. Parenteral infusion includes, for example, intramuscular, intravenous, intraarterial, intraperitoneal, or subcutaneous administration.

In the present disclosure, the term "comprise", "comprises", or "comprising" generally means including, summarizing, containing, or encompassing. In certain cases, the term also means "being" or "consisting of".

In the present disclosure, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

### GIPR-Binding Protein of the Present Disclosure

In one aspect, the present disclosure relates to a glucose-dependent insulinotropic polypeptide receptor (GIPR)-binding protein comprising at least one immunoglobulin single variable domain.

In some embodiments, the at least one immunoglobulin single variable domain may comprise a CDR1, a CDR2, and a CDR3 from a VHH set forth in any one of SEQ ID NOs: 1-7. The CDRs may be defined according to any one of the following definition systems: Kabat CDRs, AbM CDRs, Chothia CDRs, or IMGT CDRs.

In some embodiments, the at least one immunoglobulin single variable domain comprises one or more sets of CDR1, CDR2, and CDR3 selected from Table 2 below:

**Table 2. The CDRs of the VHHs of the present invention**

| Antibody No. | CDR1-3 according to Kabat | CDR1-3 according to AbM | CDR1-3 according to Chothia | CDR1-3 according to IMGT |
|---|---|---|---|---|
| iGI-9 | SEQ ID NO: 26-28 | SEQ ID NO: 29-31 | SEQ ID NO: 32-34 | SEQ ID NO: 35-37 |
| iGI-27 | SEQ ID NO: 38-40 | SEQ ID NO: 41-43 | SEQ ID NO: 44-46 | SEQ ID NO: 47-49 |
| iGI-44 | SEQ ID NO: 50-52 | SEQ ID NO: 53-55 | SEQ ID NO: 56-58 | SEQ ID NO: 59-61 |
| iGI-51 | SEQ ID NO: 62-64 | SEQ ID NO: 65-67 | SEQ ID NO: 68-70 | SEQ ID NO: 71-73 |
| iGI-72 | SEQ ID NO: 74-76 | SEQ ID NO: 77-79 | SEQ ID NO: 80-82 | SEQ ID NO: 83-85 |
| iGI-1198 | SEQ ID NO: 86-88 | SEQ ID NO: 89-91 | SEQ ID NO: 92-94 | SEQ ID NO: 95-97 |
| iGI-1225NA | SEQ ID NO: 98-100 | SEQ ID NO: 101-103 | SEQ ID NO: 104-106 | SEQ ID NO: 107-109 |

In some embodiments, the at least one immunoglobulin single variable domain comprises a CDR1, a CDR2, and a CDR3 from a VHH set forth in SEQ ID NO: 5 and/or SEQ ID NO: 6.

In some embodiments, the GIPR-binding protein of the present disclosure comprises at least one immunoglobulin single variable domain, wherein the at least one immunoglobulin single variable domain comprises one or more of the amino acid sequences set forth in SEQ ID NO: 5 and SEQ ID NOs: 8-14. "More" may be 2, 3, 4, or 5, and the more of the amino acid sequences may be identical or different.

In one specific embodiment, the at least one immunoglobulin single variable domain comprises the amino acid sequence set forth in one of SEQ ID NO: 5 and SEQ ID NOs: 8-14. For example, the GIPR-binding protein of the present disclosure comprises a sequence encompassed by the following general formula (wherein [] indicates the amino acid types that can be selected at that position):

[EQ]VQLVESGGGLVQPGGSLRLSC[AS]ASRYTLDYYAIGWFRQAPGK[EG][LR]EGVSCINS
KDGSTYYADSVKGRFTIS[KR]DNSKNTVYLQMNSLRAEDTAVYYCAAGYDPWSGIPPVQA
MCVMGYDYWGQGTLVTVSS

In some other embodiments, the GIPR-binding protein of the present disclosure comprises at least one immunoglobulin single variable domain, wherein the at least one immunoglobulin single variable domain comprises one or more of the amino acid sequences set forth in SEQ ID NO: 6 and SEQ ID NOs: 15-25.

In one specific embodiment, the at least one immunoglobulin single variable domain comprises the amino acid sequence set forth in one of SEQ ID NO: 6 and SEQ ID NOs: 15-25. For example, the GIPR-binding protein of the present disclosure comprises a sequence encompassed by the following general formula (wherein [] indicates the amino acid types that can be selected at that position):

QVQL[QV]ESGGGLVQPGGSLRLSCSASGSGFSIVAMGW[VY]RQ[AT]PGK[GQ][LR]ELVAAI
TSGGNTNYADSVKGRFTISRDN[AS]KNTIYLQMNSL[KR][AP]EDTAVYYCNADPGVVAGP
YAGMDFWGKGTTVTVSS

In some embodiments, the immunoglobulin single variable domain is a humanized VHH comprising an amino acid sequence having at least 80%, preferably at least 90%, more preferably at least 95%, and even more preferably at least 99% sequence identity to SEQ ID NO: 5 or 6. In some embodiments, the amino acid sequence of the humanized VHH comprises one or more amino acid substitutions, preferably conservative amino acid substitutions, as compared to SEQ ID NO: 5 or 6. For example, the amino acid sequence of the humanized immunoglobulin single variable domain comprises 1, 2, 3, 4, 5, 6, 7, 8, 9, or 10 conservative amino acid substitutions, as compared to SEQ ID NO: 5 or 6.

In some embodiments, the at least one immunoglobulin single variable domain may comprise one or more of the amino acid sequences set forth in SEQ ID NO: 5 and SEQ ID NOs: 8-14, and one or more of the amino acid sequences set forth in SEQ ID NO: 6 and SEQ ID NOs: 15-25, simultaneously.

In some embodiments, the at least one immunoglobulin single variable domain may comprise the amino acid sequence set forth in one of SEQ ID NO: 5 and SEQ ID NOs: 8-14 and the amino acid sequence set forth in one of SEQ ID NO: 6 and SEQ ID NOs: 15-25, simultaneously.

In some embodiments, the GIPR-binding protein of the present disclosure comprises an immunoglobulin Fc region in addition to the at least one immunoglobulin single variable domain. The inclusion of an immunoglobulin Fc region in the GIPR-binding protein of the present disclosure can cause the binding molecule to form a dimer. Fc regions that can be used in the present disclosure may be from different subtypes of immunoglobulins, for example, IgG (e.g., the IgG1, IgG2, IgG3, or IgG4 subtype), IgA1, IgA2, IgD, IgE, or IgM.

In some embodiments, a mutation may be introduced into a wild-type Fc sequence to alter an Fc-mediated related activity. The mutation includes, but is not limited to: a) a mutation that alters Fc-mediated CDC activity; b) a mutation that alters Fc-mediated ADCC activity; or c) a mutation that alters FcRn-mediated *in vivo* half-life. Such mutations are described in Leonard G Presta, Current Opinion in Immunology 2008, 20:460-470; Esohe E. Idusogie et al., J Immunol 2000, 164:4178-4184; RAPHAEL A. CLYNES et al., Nature Medicine, 2000, Volume 6, Number 4:443-446; and Paul R. Hinton et al., J Immunol, 2006, 176:346-356. For example, 1, 2, 3, 4, 5, 6, 7, 8, 9, or 10 amino acids of the CH2 region may be mutated to increase or remove Fc-mediated ADCC or CDC activity or to enhance or reduce FcRn affinity. In addition, 1, 2, 3, 4, 5, 6, 7, 8, 9, or 10 amino acids of the hinge region may be mutated to increase the stability of the protein.

In some embodiments, a mutation may be introduced into the Fc sequence to allow the mutated Fc to more easily form a homodimer or heterodimer. For example, Ridgway, Presta et al. 1996 and Carter 2001 mention a knob-hole model that utilizes the steric effect of amino acid side chain groups on the Fc contact interface, which makes it easier to form heterodimers between different Fc mutations. For example, in CN102558355A or CN103388013A, the ionic interaction force between the Fc contact interfaces is changed by changing the charges of the amino acids of the Fc contact interfaces, so that heterodimers (CN 102558355A) are more easily formed between different Fc mutation pairs, or homodimers (CN 103388013A) are more easily formed between Fc with the same mutation.

The immunoglobulin Fc region is preferably a human immunoglobulin Fc region, for example, a human IgG1, IgG2, IgG3, or IgG4 Fc region. In some specific embodiments, the amino acid sequence of the immunoglobulin Fc region is set forth in SEQ ID NO: 110 or SEQ ID NO: 113.

In some specific embodiments, in the GIPR-binding protein of the present disclosure, the immunoglobulin Fc region (e.g., human IgG1 Fc region) is linked directly or linked indirectly by a linker to the C-terminus of the immunoglobulin single variable domain (e.g., VHH).

In some embodiments, the GIPR-binding protein of the present disclosure comprises one immunoglobulin single variable domain that specifically binds to GIPR, wherein the immunoglobulin single variable domain that specifically binds to GIPR is linked directly or indirectly to an immunoglobulin Fc region that allows the GIPR-binding protein to form a dimeric molecule comprising two GIPR-binding domains. Such a GIPR-binding protein is also referred to as a bivalent GIPR-binding protein. In some embodiments, the dimer is a homodimer.

In some embodiments, the GIPR-binding protein of the present disclosure comprises two immunoglobulin single variable domains that specifically bind to GIPR and one immunoglobulin Fc region that are linked directly or linked indirectly by a linker to each other, wherein the immunoglobulin Fc region allows the GIPR-binding protein to form a dimeric molecule comprising four GIPR-binding domains. Such a GIPR-binding protein is also referred to as a tetravalent GIPR-binding protein. In some embodiments, the dimer is a homodimer. In some embodiments, the two immunoglobulin single variable domains that specifically bind to GIPR in the GIPR-binding protein bind to different regions or different epitopes of GIPR, respectively.

In the present disclosure, multiple immunoglobulin single variable domains may be linked directly or linked indirectly by a linker, and an immunoglobulin single variable domain and an immunoglobulin Fc region may be linked directly or linked indirectly by a linker. The linker may be a non-functional amino acid sequence that is 1-20 or more amino acids in length and has no secondary or higher-order structure. For example, the linker is a flexible linker, such as one or more of (GGG)ₙ, (GGGS)ₙ, (GGGA)ₙ, (GGGAA)ₙ, and (GGGGS)ₙ, wherein n is an integer selected from 1 to 30, an integer selected from 1 to 20, an integer selected from 1 to 10, an integer selected from 1 to 9, an integer selected from 1 to 8, an integer selected from 1 to 7, an integer selected from 1 to 6, an integer selected from 1 to 5, an integer selected from 1 to 4, or an integer selected from 1 to 3. In some embodiments, the linker is GGGGS, GS, GAP, (GGGGS)x3, or the like.

In some embodiments, the GIPR-binding protein of the present disclosure has at least one of the following characteristics:
(a) having specific binding to human GIPR;
(b) binding to human GIPR with a KD value of less than 1 × 10⁻⁷ M, preferably less than 1 × 10⁻⁸ M;
(c) blocking the interaction of GIP with GIPR; and
(d) being capable of reducing the body weight and/or blood glucose of a subject.

### Fusion Protein

In another aspect, the present disclosure further relates to a fusion protein comprising the GIPR-binding protein described in the present disclosure.

In some embodiments, the fusion protein comprises, in addition to the GIPR-binding protein, another or more biologically active proteins that may be any protein with biological, therapeutic, prophylactic, or diagnostic significance or function and, when administered to a subject, mediate biological activity that can prevent or alleviate a disease, disorder, or condition. Specifically, the biologically active proteins may be agonists, antagonists, modulators, ligands, cytokines, enzymes, or hormones, particularly biologically active proteins that can be used for the prevention and/or treatment of metabolic diseases, especially obesity, overweight, and/or diabetes.

### Multispecific Antibody

In another aspect, the present disclosure further relates to a multispecific antibody comprising the GIPR-binding protein described in the present disclosure.

In some embodiments, the multispecific antibody comprises, in addition to the GIPR-binding protein, one or more additional antigen-binding regions that, compared to the GIPR-binding protein and/or the fusion protein, bind to a different antigen or a different epitope of the same antigen.

### Nucleic Acid, Vector, and Host cell

In another aspect, the present disclosure relates to a nucleic acid molecule encoding the GIPR-binding protein or fusion protein of the present disclosure. The nucleic acid of the present disclosure may be an RNA, DNA, or cDNA. According to one embodiment of the present disclosure, the nucleic acid of the present disclosure is a substantially isolated nucleic acid.

The nucleic acid of the present disclosure may also be in the form of a vector, such as a plasmid, cosmid, or YAC, may be present in the vector, and/or may be part of the vector. The vector may especially be an expression vector, i.e., a vector that can provide the *in vitro* and/or *in vivo* (i.e., in a suitable host cell, host organism, and/or expression system) expression of the GIPR-binding protein. The expression vector generally comprises at least one nucleic acid of the present disclosure that is operably linked to one or more suitable expression control elements (e.g., promoters, enhancers, and terminators). The selection of the elements and sequences thereof for expression in a particular host is common knowledge of those skilled in the art. Specific examples of control elements and other elements useful or necessary for the expression of the GIPR-binding protein of the present disclosure include, for example, promoters, enhancers, terminators, integration factors, selection markers, leader sequences, and reporter genes.

The nucleic acid of the present disclosure may be prepared or obtained by known means (e.g., by automated DNA synthesis and/or recombinant DNA techniques) based on information about the amino acid sequence of the polypeptide of the present disclosure given herein, and/or may be isolated from a suitable natural source.

In another aspect, the present disclosure relates to a recombinant host cell expressing or capable of expressing one or more GIPR-binding proteins or fusion proteins of the present disclosure and/or comprising the nucleic acid or vector of the present disclosure. The preferred host cells of the present disclosure are bacterial cells, fungal cells, or mammalian cells.

Suitable bacterial cells include cells of Gram-negative bacterial strains (e.g., *Escherichia coli* strains, Proteus strains, and Pseudomonas strains) and Gram-positive bacterial strains (e.g., Bacillus strains, Streptomyces strains, Staphylococcus strains, and Lactococcus strains).

Suitable fungal cells include cells of species of Trichoderma, Neurospora, and Aspergillus, or include cells of species of Saccharomyces (e.g., *Saccharomyces cerevisiae*), Schizosaccharomyces (e.g., *Schizosaccharomyces pombe*), Pichia (e.g., *Pichia pastoris* and *Pichia methanolica*), and Hansenula. Suitable mammalian cells include, for example, HEK293 cells, CHO cells, BHK cells, HeLa cells, COS cells, or the like.

However, known cells, such as amphibian cells and insect cells, and any other cells used in the art for the expression of heterologous proteins may also be used in the present disclosure.

The GIPR-binding protein or fusion protein of the present disclosure may be produced intracellularly (e.g., in the cytoplasm, in the periplasm, or in inclusion bodies) in a cell as described above, followed by isolation from the host cell and optionally further purification; or it may be produced extracellularly (e.g., in a culture medium in which the host cell is cultured), followed by isolation from the culture medium and optionally further purification.

Methods and reagents for recombinant production of polypeptides, e.g., specifically suitable expression vectors, transformation or transfection methods, selection markers, methods of inducing protein expression, culture conditions, etc., are known in the art. Similarly, protein isolation and purification techniques suitable for use in the methods for producing the GIPR-binding protein of the present disclosure are well known to those skilled in the art.

### Pharmaceutical Composition

In another aspect, the present disclosure provides a composition, e.g., a pharmaceutical composition, comprising one of or a combination of the GIPR-binding protein, fusion protein, multispecific antibody, or conjugate of the present disclosure formulated together with a pharmaceutically acceptable carrier.

As used herein, "pharmaceutically acceptable carrier" includes any and all physiologically compatible solvents, dispersion media, coatings, antibacterial and antifungal agents, buffers, stabilizers, isotonic agents, absorption delaying agents, and the like. Preferably, the carrier is suitable for intravenous, intramuscular, subcutaneous, parenteral, spinal, or epidermal administration (e.g., by injection or infusion). Depending on the route of administration, the active compound, i.e., the antibody molecule, may be encapsulated in a material to protect the compound from acids and other natural conditions that may inactivate the compound.

The amount of the active ingredient that can be combined with a carrier material to prepare a single dosage form varies depending on the subject of interest and the particular mode of administration. The amount of the active ingredient that can be combined with a carrier material to prepare a single dosage form is generally an amount of the composition that produces a therapeutic effect. Generally, such amounts range from about 0.01% to about 99% of the active ingredient, for example, from about 0.1% to about 70% or from about 1% to about 30% of the active ingredient, based on 100%, in combination with a pharmaceutically acceptable carrier.

Actual dosage levels of the active ingredient in the pharmaceutical composition of the present disclosure may be varied so as to obtain an amount of the active ingredient that is effective in achieving the desired therapeutic response for a particular patient, composition, and mode of administration, without being toxic to the patient. The selected dosage level depends upon a variety of pharmacokinetic factors, including the activity of the particular composition of the present disclosure employed, or an ester, salt, or amide thereof, the route of administration, the time of administration, the rate of excretion of the particular compound employed, the duration of treatment, other drugs, compounds, and/or materials used in combination with the particular composition employed, the age, sex, body weight, condition, general health, and medical history of the patient of interest, and similar factors well known in the medical arts.

The composition of the present disclosure may be administered via one or more routes of administration using one or more methods well known in the art. It will be appreciated by those skilled in the art that the route and/or mode of administration varies depending on the desired result. The preferred routes of administration of the GIPR-binding protein of the present disclosure include intravenous, intramuscular, intradermal, intraperitoneal, subcutaneous, spinal, or other parenteral routes of administration, such as injection or infusion. The phrase "parenteral administration" as used herein refers to a mode of administration other than enteral and topical administrations, generally injection, including but not limited to intravenous, intramuscular, intraarterial, intramembranous, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion.

### Prevention and/or Treatment of Disease

In another aspect, the present disclosure further provides a method for preventing and/or treating a disease, particularly for treating and/or preventing a metabolic disease, comprising administering to a subject in need thereof an effective amount of the GIPR-binding protein, fusion protein, multispecific antibody, conjugate, or pharmaceutical composition of the present disclosure. The metabolic disease includes obesity, overweight, and/or diabetes.

In some embodiments, in the process of preventing and/or treating the metabolic disease, in addition to the GIPR-binding protein, fusion protein, multispecific antibody, conjugate, or pharmaceutical composition of the present disclosure, an additional therapeutic drug, such as an insulin (including porcine or bovine insulin, human insulin, insulin aspart, insulin lispro, isophane insulin, insulin glargine, insulin detemir, LY3209590, etc.), a biguanide (such as metformin, etc.), a thiazolidinedione (including pioglitazone, rosiglitazone, etc.), a sulfurylurea (including glipizide, gliclazide, glibenclamide, glibornuride, gliquidone, glisoxepide, glimepiride, etc.), a phenylalanine (including repaglinide, nateglinide, mitiglinide, etc.), an α-glucosidase inhibitor (including acarbose, voglibose, etc.), a DPP4 inhibitor (including sitagliptin, linagliptin, gosogliptin, vildagliptin, saxagliptin, alogliptin, gemigliptin, teneligliptin, etc.), an SGLT2 inhibitor (including dapagliflozin, canagliflozin, empagliflozin, etc.), orlistat, a GCGR agonist, a GLP-1R agonist, or the like may be further administered to the subject in need thereof.

The GLP-1R agonist may be GLP-1(7-37), GLP-1(7-36)-NH2, liraglutide, albiglutide, lixisenatide, dulaglutide, semaglutide, exendin-4, exendin-3, PEG-loxenatide, tirzepatide, GMA102, PB119, albenatide, mazdutide, supaglutide, cotadutide, danuglipron, HM-15211, efinopegdutide, or the like. The additional therapeutic drug and the binding protein/fusion protein/multispecific antibody/conjugate/pharmaceutical composition of the present disclosure may be administered simultaneously or sequentially; when the two are administered sequentially, the interval between the administrations of the two is no more than 1 week, preferably no more than 3 days, more preferably no more than 2 days, and even more preferably no more than 1 day.

In some other embodiments, the present disclosure further provides use of the GIPR-binding protein, fusion protein, multispecific antibody, conjugate, or pharmaceutical composition of the present disclosure in the manufacture of a medicament for treating and/or preventing a metabolic disease. In some other embodiments, the present disclosure provides a GIPR-binding protein, a fusion protein, a multispecific antibody, a conjugate, or a pharmaceutical composition for use in the treatment and/or prevention of a metabolic disease.

In some other embodiments, the present disclosure provides a method for detecting the presence and/or determining the content of a GIPR protein, comprising providing the GIPR-binding protein and/or the fusion protein and/or the multispecific antibody and/or the conjugate. For example, the method may be an *ex vivo* or *in vitro* method. For example, the method may be a method for non-therapeutic purposes.

In some embodiments, the present disclosure provides a method for detecting the presence of a GIPR protein in a sample, comprising contacting the sample with the GIPR-binding protein and/or the fusion protein and/or the multispecific antibody and/or the conjugate, wherein the binding of the GIPR-binding protein and/or the fusion protein and/or the multispecific antibody and/or the conjugate to the GIPR protein is indicative of the presence of the GIPR protein in the sample.

In some embodiments, the present disclosure provides a method for determining the content of a GIPR protein in a sample, comprising contacting the sample with the GIPR-binding protein and/or the fusion protein and/or the multispecific antibody and/or the conjugate, wherein the amount of the binding of the GIPR-binding protein and/or the fusion protein and/or the multispecific antibody and/or the conjugate to the GIPR protein is indicative of the content of the GIPR protein in the sample. The present invention further provides use of the GIPR-binding protein, fusion protein, multispecific antibody, or conjugate of the present disclosure in the manufacture of a kit for detecting the presence and/or determining the content of a GIPR protein in a sample.

The sample may be a sample of blood, plasma, amniotic fluid, or the like.

In another aspect, the present disclosure provides a kit that may comprise the GIPR-binding protein, fusion protein, multispecific antibody, conjugate, and/or pharmaceutical composition described in the present disclosure. It may comprise the GIPR-binding protein, fusion protein, multispecific antibody, conjugate, and/or pharmaceutical composition described in the present disclosure in a single common container, or may be optionally combined with one or more therapeutic agents, and optionally formulated together in a pharmaceutical composition.

In another aspect, the present disclosure provides an administration apparatus that can be used to administer the GIPR-binding protein, fusion protein, multispecific antibody, conjugate, and/or pharmaceutical composition described in the present disclosure.

In another aspect, the present disclosure provides the following embodiments:
1. A glucose-dependent insulinotropic polypeptide receptor (GIPR)-binding protein, comprising at least one immunoglobulin single variable domain, wherein the at least one immunoglobulin single variable domain comprises a CDR1, a CDR2, and a CDR3 from a VHH set forth in SEQ ID NO: 5 and/or SEQ ID NO: 6.
2. The GIPR-binding protein according to embodiment 1, wherein the CDRs are defined according to the following definition system: Kabat, AbM, Chothia, or IMGT.
3. The GIPR-binding protein according to embodiment 1, wherein the CDR1, CDR2, and CDR3 from the VHH set forth in SEQ ID NO: 5 are selected from any one of the following groups: SEQ ID NOs: 74-76, SEQ ID NOs: 77-79, SEQ ID NOs: 80-82, and SEQ ID NOs: 83-85.
4. The GIPR-binding protein according to embodiment 3, wherein the at least one immunoglobulin single variable domain comprises one or more of the amino acid sequences set forth in SEQ ID NO: 5 and SEQ ID NOs: 8-14.
5. The GIPR-binding protein according to embodiment 4, wherein the at least one immunoglobulin single variable domain comprises the amino acid sequence set forth in one of SEQ ID NO: 5 and SEQ ID NOs: 8-14.
6. The GIPR-binding protein according to embodiment 1, wherein the CDR1, CDR2, and CDR3 from the VHH set forth in SEQ ID NO: 6 are selected from any one of the following groups: SEQ ID NOs: 86-88, SEQ ID NOs: 89-91, SEQ ID NOs: 92-94, and SEQ ID NOs: 95-97.
7. The GIPR-binding protein according to embodiment 6, wherein the at least one immunoglobulin single variable domain comprises one or more of the amino acid sequences set forth in SEQ ID NO: 6 and SEQ ID NOs: 15-25.
8. The GIPR-binding protein according to embodiment 7, wherein the at least one immunoglobulin single variable domain comprises the amino acid sequence set forth in one of SEQ ID NO: 6 and SEQ ID NOs: 15-25.
9. The GIPR-binding protein according to any one of embodiments 1-8, wherein the at least one immunoglobulin single variable domain comprises the amino acid sequence set forth in one of SEQ ID NO: 5 and SEQ ID NOs: 8-14 and/or the amino acid sequence set forth in one of SEQ ID NO: 6 and SEQ ID NOs: 15-25.
10. The GIPR-binding protein according to any one of embodiments 1-9, further comprising an immunoglobulin Fc region, preferably a human immunoglobulin Fc region, and more preferably a human IgG1, IgG2, IgG3, or IgG4 Fc region.
11. The GIPR-binding protein according to embodiment 10, wherein the amino acid sequence of the immunoglobulin Fc region is set forth in SEQ ID NO: 110 or SEQ ID NO: 113.
12. The GIPR-binding protein according to embodiment 10 or 11, wherein the immunoglobulin Fc region is linked directly or linked indirectly by a linker to the at least one immunoglobulin single variable domain.
13. The GIPR-binding protein according to any one of embodiments 1-12, having at least one of the following characteristics:
   (a) having specific binding to human GIPR;
   (b) binding to human GIPR with a K_{D} value of less than 1 × 10⁻⁷ M, preferably less than 1 × 10⁻⁸ M;
   (c) blocking the interaction of GIP with GIPR; and
   (d) being capable of reducing the body weight and/or blood glucose of a subject.
14. A fusion protein, comprising the GIPR-binding protein according to any one of embodiments 1-13.
15. A conjugate, comprising the GIPR-binding protein according to any one of embodiments 1-13 and/or the fusion protein according to embodiment 14.
16. A nucleic acid molecule, wherein the nucleic acid molecule encodes the GIPR-binding protein according to any one of embodiments 1-13 and/or the fusion protein according to embodiment 14.
17. An expression vector, comprising the nucleic acid molecule according to embodiment 15 operably linked to an expression control element.
18. A recombinant cell, comprising the nucleic acid molecule according to embodiment 16 and/or transformed with the expression vector according to embodiment 17 and capable of expressing the GIPR-binding protein or the fusion protein comprising the GIPR-binding protein.
19. A pharmaceutical composition, comprising the GIPR-binding protein according to any one of embodiments 1-13, the fusion protein according to embodiment 14, and/or the conjugate according to embodiment 15, and a pharmaceutically acceptable carrier.
20. A kit, comprising the GIPR-binding protein according to any one of embodiments 1-13, the fusion protein according to embodiment 14, the conjugate according to embodiment 15, and/or the pharmaceutical composition according to embodiment 19.
21. A method for determining the presence and/or content of a GIPR protein and/or inhibiting binding of a GIPR protein to a ligand thereof, comprising providing the GIPR-binding protein according to any one of embodiments 1-13, the fusion protein according to embodiment 14, and/or the conjugate according to embodiment 15.
22. A method for treating and/or preventing a metabolic disease and/or symptom, comprising administering to a subject in need thereof an effective amount of the GIPR-binding protein according to any one of embodiments 1-13, the fusion protein according to embodiment 14, the conjugate according to embodiment 15, or the pharmaceutical composition according to embodiment 18.
23. The method according to embodiment 22, wherein the metabolic disease and/or symptom is obesity, overweight, and/or diabetes.

Without being bound by any theory, the following examples are intended only to illustrate various technical solutions of the invention of the present disclosure rather than to limit the scope of the invention of the present disclosure.

### Examples

### Example 1

### Screening for GIPR Heavy-Chain Single-Domain Antibodies

### 1.1. Library construction

Before immunization, 50 mL of arterial blood was collected from an alpaca into a vacuum blood collection tube, and the supernatant was collected as pre-immunization serum. A healthy alpaca was selected and immunized with an antigen containing human GIPR via multi-point intramuscular injection in the neck once every two weeks, and a total of six immunizations were administered. At the end of the final immunization, 50 mL of arterial blood was collected from the alpaca into a vacuum blood collection tube, and the supernatant was collected as post-immunization serum. Lymphocytes were isolated using density gradient centrifugation, and total RNA was extracted using an RNA extraction kit provided by QIAGEN. The extracted RNA was all reverse-transcribed into cDNA using the Super-Script III FIRST STRANDSUPERMIX kit according to the instructions. Nested PCR was then performed to amplify nucleic acid fragments encoding the variable regions of heavy-chain antibodies.

Nucleic acid fragments of the target heavy-chain single-domain antibody were recovered and cloned into the phage display vector pComb3XSS using the restriction endonuclease *SfiI.* The product was subsequently electroporated into TG1 electrocompetent E. coli cells to construct an anti-GIPR immune single-domain antibody phage display library, and the library was identified. The size of the library was calculated to be 1.69 × 10⁹ through gradient dilution plating. To determine the insertion rate of the library, 50 randomly selected clones were sequenced. The results showed that all of the 50 clones contained the correct exogenous fragment insertion-the correct rate was 100%. DNA and amino acid sequence analysis and alignment of the sequenced clones confirmed that all sequences were alpaca VHH sequences, with an estimated diversity of more than 95%.

### 1.2. Panning for GIPR heavy-chain single-domain antibodies

The phage library obtained in Example 1.1 was subjected to panning. Bound phages were obtained through screening using the proteins GIPR-ECD-muFc (a mouse Fc-fused human GIPR extracellular region fragment), GIPR-ECD-chis (a his tag-fused human GIPR extracellular region fragment), and GIPR-ECD-laFc (an alpaca Fc-fused human GIPR extracellular region fragment). Meanwhile, negative panning was performed using Control-1 (C1), a muFc-containing fusion protein, and Control-2 (C2), a laFc-containing fusion protein, to remove non-specifically bound phages.

The binding-positive phages obtained after the panning were used to infect blank E. coli cells, which were then plated. Subsequently, colonies were picked, inoculated separately into 2TY-AG (containing 10% glycerol), and left to stand overnight at room temperature. The next day, the cultures were separately inoculated into 200 µL of 2TY-AG, with an inoculation amount of 1%, and incubated at 37 °C at 250 rpm until OD600 reached about 0.5. Helper phage M13KO7 was added for infection (at a multiplicity of infection of 1:20). The cultures were left to stand at 37 °C for 15 min and then incubated at 220 rpm for 45 min. 800 µL of 2TY-AG was added to each well, and the plate was incubated overnight at 30 °C at 220 rpm and centrifuged the next day. The supernatants were collected for ELISA assays. Plates were coated overnight at 4 °C with GIPR-ECD-Chis, GIPR-ECD-laFc, C1, and C2, respectively, and the obtained supernatants were added. The plates were incubated at room temperature for 2 hours. After washing, the secondary antibody Goat anti-HA tag HRP (purchased from Abcam) was added, and the plates were incubated at room temperature for 2 hours. After washing, TMB chromogenic solution was added, and absorbance values at wavelengths of 450 nm and 650 nm were read. The final absorbance values were obtained by subtracting the absorbance values at the wavelength of 650 nm from the absorbance values at the wavelength of 450 nm. Among the 297 clones obtained through panning, there were 191 positive clones with OD values of >1.0. Some of the positive clones with specific binding to GIPR-ECD-laFc are shown in Table 3. The above positive clones with specific binding were sequenced to obtain antibody sequences.

**Table 3. Positive clones with specific binding to GIPR-ECD-laFc**

| Candidate clone | OD value | | |
|---|---|---|---|
| | GIPR-ECD-laFc | C1 | C2 |
| iGI-107 | 2.322 | 0.022 | 0.023 |
| iGI-44 | 2.313 | 0.018 | 0.023 |
| iGI-104 | 2.275 | 0.019 | 0.027 |
| iGI-27 | 2.225 | 0.018 | 0.023 |
| iGI-79 | 2.224 | 0.017 | - |
| iGI-88 | 2.224 | 0.019 | - |
| iGI-9 | 2.211 | 0.018 | 0.028 |
| iGI-86 | 2.211 | 0.016 | - |
| iGI-36 | 2.203 | 0.017 | 0.024 |
| iGI-64 | 2.203 | 0.064 | - |
| iGI-72 | 2.196 | 0.019 | 0.077 |
| iGI-51 | 2.169 | 0.042 | 0.027 |
| iGI-1146 | 2.04 | - | 0.04 |
| iGI-1213 | 2.1 | - | 0.032 |
| iGI-1205NA | 1.917 | - | 0.026 |
| iGI-1225NA | 1.912 | - | 0.035 |
| iGI-1198 | 1.947 | - | 0.027 |
| iGI-1050 | 1.986 | - | 0.028 |
| iGI-1122 | 1.923 | - | 0.024 |
| iGI-1069 | 1.987 | - | 0.031 |

### Example 2

### Preparation of Fc Fusion Proteins of GIPR Single-Domain Antibodies Using Mammalian Cells

### 2.1. Preparation of plasmids expressing GIPR single-domain antibody-Fc fusion proteins

Primers were designed and used to PCR-amplify the GIPR single-domain antibody VHH fragments, and the fragments were fused with a DNA fragment encoding human IgG1-Fc (amino acid sequence SEQ ID NO: 110) and cloned into a conventional mammalian expression vector, yielding recombinant plasmids for expressing GIPR single-domain antibody-Fc fusion proteins in mammals. Universal primers were used to amplify different VHH fragments. The universal primers were as follows:
Upstream primer cccACCGGTCAGGTGCAGCTGCAGGAGTC (SEQ ID NO: 111)
Downstream primer cccGGATCCTGAGGAGACGGTGACCTGG (SEQ ID NO: 112)

### 2.2. Preparation of Fc fusion proteins of GIPR single-domain antibodies

The plasmid vectors constructed in 2.1 were transfected into HEK293 cells for transient antibody expression. The recombinant expression plasmids were diluted with Freestyle293 culture medium and added to a PEI (polyethylenimine) solution required for transformation. Each plasmid/PEI mixture was added to a suspension of HEK293 cells, and the cells were cultured under suspension conditions at 37 °C with 5% CO₂. After 5-6 days of culture, the transient expression culture supernatants were collected and purified by Protein A affinity chromatography, yielding the target GIPR single-domain antibody-Fc fusion proteins. The purities of the proteins were tested by SDS-PAGE. The expression levels of the proteins are shown in Table 4, and after single-step purification, the SDS purities of the proteins were all greater than 95%.

**Table 4. The expression levels of Fc fusion proteins of GIPR single-domain antibodies**

| Antibody | Expression level mg/L |
|---|---|
| iGI-9-Ld-Fc | 98 |
| iGI-27-Ld-Fc | 71 |
| iGI-44-Ld-Fc | 180 |
| iGI-51-Ld-Fc | 173 |
| iGI-72-Ld-Fc | 128 |
| iGI-1146-Ld-Fc | 175 |
| iGI-1213-Ld-Fc | 200 |
| iGI-1205NA-Ld-Fc | 272 |
| iGI-1225NA-Ld-Fc | 27 |
| iGI-1198-Ld-Fc | 296 |
| iGI-1050-Ld-Fc | 286 |
| iGI-1122-Ld-Fc | 330 |
| iGI-1069-Ld-Fc | 311 |
| iGI-1213n1-Ld-Fc | 84 |
| iGI-1213n2-Ld-Fc | 259 |
| iGI-1213n3-Ld-Fc | 263 |
| iGI-1213n4-Ld-Fc | 25 |

### Example 3

### Identification of Functions of GIPR Single-Domain Antibodies-Fe Fusion Proteins

### 3.1. Abilities of GIPR single-domain antibody-Fc fusion proteins to bind to GIPR

293T-GIPR cells (HEK293T cells expressing human GIPR) were plated in a cell culture plate, and GIPR single-domain antibody-Fc fusion protein samples were added, with two final concentrations of 100 µg/mL and 10 µg/mL. The anti-human IgG-APC secondary antibody was added, followed by incubation. The mean fluorescence intensity (MFI) was measured using a fluorescence-activated cell sorter. The results are shown in Table 5.

**Table 5. The abilities of Fc fusion proteins of GIPR single-domain antibodies to bind to GIPR**

| Antibody | 293T-GIPR | |
|---|---|---|
| | 100 µg/mL | 10 µg/mL |
| iGl-9-Ld-Fc | 6498 | 6324 |
| iGI-27-Ld-Fc | 217 | 190 |
| iGI-44-Ld-Fc | 6289 | 6583 |
| iGI-51-Ld-Fc | 5520 | 5963 |
| iGI-72-Ld-Fc | 6961 | 5135 |
| iGI-151-Ld-Fc | 297 | 183 |
| iGI-185-Ld-Fc | 224 | 173 |
| Negative | 152 | - |
| Black | 169 | - |

### 3.2. Neutralizing activity of GIPR single-domain antibody-Fc fusion proteins

(1) GIP-C12H4-Fc4 (human GIP-IgG4 Fc fusion protein) and GIPR single-domain antibody-Fc samples (25 µL) were separately added to a 96-well plate. Then 293T-GIPR cells were added. After incubation, an assay was performed using a cAMP kit, and the absorbance at 450 nm/550 nm was measured using a microplate reader. The results are shown in Table 6.

**Table 6. The neutralizing activity of GIPR single-domain antibody-Fc fusion proteins**

| MEAN cAMP expression level (pM) | Amount of fusion protein | iGI-9-Ld-Fc | iGI-44-Ld-Fc | iGI-51-Ld-Fc | iGI-72-Ld-Fc | iGI-229-Ld-Fc | Negative (without antibody addition) |
|---|---|---|---|---|---|---|---|
| + Experimental culture medium | 10 µg/mL | 2.793 | 3.087 | 3.1585 | 2.85 | 3.3575 | 4.00775 |
| | 1 µg/mL | 4.9995 | 3.731 | 3.6805 | 3.3055 | 3.6585 | - |
| | 0.1 µg/m | 4.084 | 3.8895 | 3.15 | 3.215 | 3.9085 | - |
| +100 ng/mL GIP-C12H4-Fc4 | 10 µg/mL | 113.7515 | 122.317 | 136.7475 | 5.7855 | 139.393 | 111.5675 |
| | 1 µg/mL | 124.824 | 113.9625 | 109.424 | 52.6745 | 108.952 | - |
| | 0.1 µg/mL | 92.8595 | 130.5825 | 112.3975 | 118.553 | 132.348 | - |

(2) GIP-C12H4-Fc4 and GIPR single-domain antibody-Fc samples (25 µL) were separately added to a 96-well plate, and 293T-GIPRFL-GFP-PURO-5 cells, which are 293T cells that stably express full-length human GIPR protein on the cell membrane, were then added. After incubation, an assay was performed using a cAMP kit. The RLU values were read, and the neutralizing activity (%) was calculated. The results are shown in Table 7.

**Table 7. The neutralizing activity of GIPR single-domain antibody-Fc fusion proteins**

| Protein concentration | Neutralizing activity (%) | | | |
|---|---|---|---|---|
| | iGI-1122-Ld-Fc | iGI-1198-Ld-Fc | iGI-1213n1-Ld-Fc | iGI-72-Ld-Fc |
| 10 µg/mL | 66.89 | 69.80 | 71.16 | 72.46 |
| 1 µg/mL | 0.00 | -4.71 | 48.71 | 62.85 |
| 0.1 µg/mL | 0.00 | -4.71 | -14.82 | 2.28 |
| 0.01 µg/mL | -4.71 | 14.82 | -17.49 | 0.00 |

### 3.3. Assay for affinities of GIPR single-domain antibody-Fc fusion proteins

The binding kinetic profiles of GIPR single-domain antibody-Fc fusion proteins to GIPR-ECD-chis were assessed using biolayer interferometry (BLI). The GIPR single-domain antibody-Fc fusion proteins were directly immobilized onto AHC sensors. Then GIPR-ECD-chis was diluted to seven concentrations and allowed to associate with the immobilized GIPR single-domain antibody-Fc fusion proteins. The equilibrium dissociation constants (KD), association rates (Kₐ), and dissociation rates (K_{dis}) were calculated using Octet K2 data analysis software 9.0. The results are shown in Tables 8 and 9.

**Table 8. The affinities of GIPR single-domain antibody-Fc fusion proteins**

| Antibody | KD (M) | Kₐ (1/Ms) | K_{dis} (1/s) |
|---|---|---|---|
| iGI-72-Ld-Fc | 1.616E-10 | 1.957E+05 | 3.163E-05 |
| iGI-1122-Ld-Fc | 4.575E-10 | 2.494E+05 | 1.141E-04 |
| iGI-1146-Ld-Fc | 3.236E-9 | 1.368E+05 | 4.551E-04 |

**Table 9. The affinities of GIPR single-domain antibody-Fc fusion proteins**

| Antibody | KD (M) | Kₐ (1/Ms) | K_{dis} (1/s) |
|---|---|---|---|
| iGI-1198-Ld-Fc | 1.86E-09 | 8.39E+04 | 1.56E-04 |
| iGI-72-Ld-Fc | 5.90E-10 | 1.47E+05 | 8.69E-05 |

### 3.4. Assay for blocking activity of GIPR single-domain antibody-Fc fusion protein

A plate was coated with 5 µg/mL mouse GIPR-Chis protein and blocked with BSA, and serially diluted AMG-GIPR-mab2 and iGI72-Ld-Fc were then added and well incubated. The diluent contained 1.2 µg/mL GIP-C12H4-Fc4. Then, Mouse Anti-Human IgG4 pFc' [HP6023] (HRP) (Cat. No. ab99817), diluted 1:2000, was added. After TMB color development, the plate was analyzed using a microplate reader. The results are shown in FIG. 1.

The positive control AMG-GIPR-mab2 was prepared by synthesizing the gene of the sequence in MAbs. 2020 Jan-Dec; 12(1): 1710047 and performing transient expression in 293 cells using the method described above.

### 3.5. Assay for non-specific binding of GIPR single-domain antibody-Fc fusion proteins to null cells

CHOK1 null cells were resuspended in 3% BSA-PBS. The final concentrations of GIPR single-domain antibody-Fc fusion proteins were set to 5 µg/mL and 50 µg/mL. Meanwhile, a negative control and a blank control were set. After washing, the secondary antibody APC anti-human IgG Fc was added. After washing, the cells were resuspended in PBS-BSA Buffer and assayed using a flow cytometer. The results are shown in Table 10: iGI-72-Ld-Fc and iGI-1198-Ld-Fc did not exhibit non-specific binding, while iGI-1225-Ld-Fc and iGI-1225NA-Ld-Fc exhibited non-specific binding.

**Table 10. The non-specific binding of GIPR single-domain antibody-Fc fusion proteins to null cells**

| Antibody | Results | | | |
|---|---|---|---|---|
| | 50 µg/mL | 5 µg/mL | Blank | Negetive |
| iGI-1225-Ld-Fc | 2325 | 558 | 256 | 263 |
| iGI-1198-Ld-Fc | 263 | 263 | | |
| iGI-1225NA-Ld-Fc | 1469 | 306 | | |
| iGI-72-Ld-Fc | 259 | 260 | | |

### 3.6. Study of in vivo efficacy of GIPR single-domain antibody-Fc fusion protein

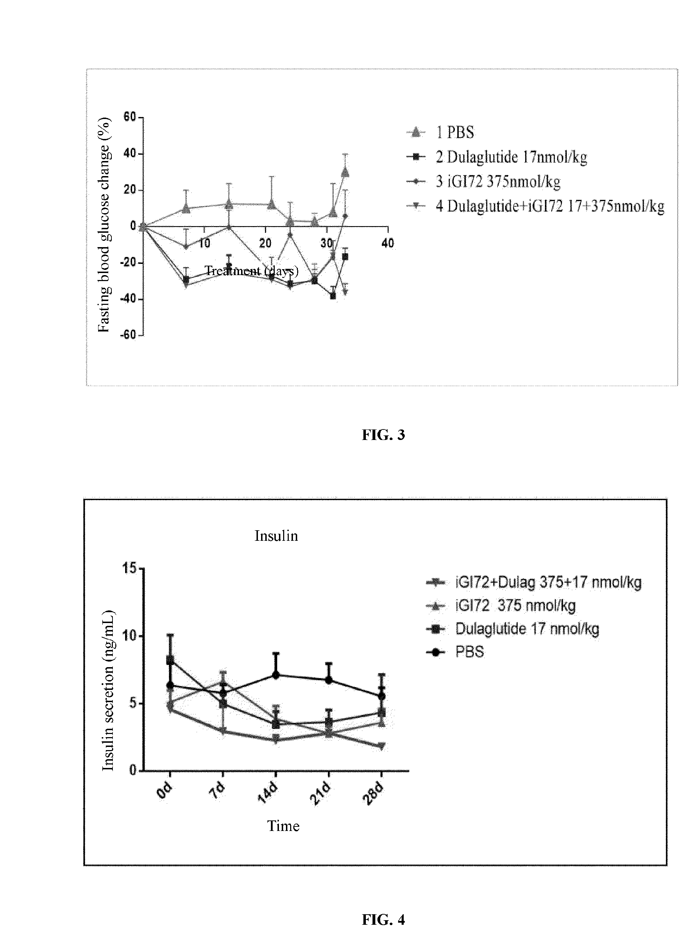

After 16 DIO mice were fasted overnight at 16 weeks of age, their blood glucose levels and body weights were measured, and they were randomized into 4 groups. The day of grouping and administration is denoted by D0. PBS, dulaglutide 17 nmol/kg, iGI-72-Ld-Fc 375 nmol/kg, and dulaglutide 17 nmol/kg + iGI-72-Ld-Fc 17+375 nmol/kg were separately administered twice weekly and 10 times in total. After administration, general clinical observations were conducted once weekly, the body weight was measured twice weekly, and the fasting blood glucose and fasting insulin levels were measured once weekly. The results are shown in FIGs. 2 to 5: The iGI-72-Ld-Fc treatment group exhibited significant reductions in the body weight of DIO mice and improvements in fasting glucose, fasting insulin, and insulin resistance HOMA-IR. The iGI-72-Ld-Fc + dulaglutide treatment group exhibited further enhanced efficacy, demonstrating a synergistic effect.

### Example 4

### Humanization of GIPR Single-Domain Antibodies

The humanization was accomplished using protein surface amino acid humanization (resurfacing) and VHH humanized universal framework grafting (CDR grafting to a universal framework).

First, the universal humanized VHH framework hNbBcII10FGLA (PDB No. 3EAK), designed by Cécile Vincke et al. based on sequence homology, was obtained. The framework design is based on the nanobody NbBcII10 (PDB No. 3DWT). Modeling was performed using Modeller9, and the relative solvent accessibility of amino acids on the framework was calculated based on the three-dimensional structure of the protein.

The specific steps of VHH humanized universal framework grafting are as follows: A highly homologous human antibody sequence is obtained through IMGT, and the target sequence is humanized with reference to the universal humanized VHH framework hNbBcII10FGLA (PDB No. 3EAK). The framework of the highly homologous sequence is used as a framework template, and the CDRs are replaced with the CDRs of the target antibody. Non-surface amino acids on the framework are then back-mutated based on the model to complete the humanization of the target antibody. Antibodies iGI-72 and iGI-1198 were humanized, yielding 8 and 11 humanized huGI variants of the antibodies, respectively.

### Example 5

### Preparation of Fc Fusion Proteins of Humanized Single-Domain Antibodies

### 5.1. Preparation of Fc fusion plasmids of humanized single-domain antibodies

The genes of the humanized sequences in Example 4 were synthesized, fused with a DNA fragment encoding human IgG1-Fc, and cloned into a conventional mammalian expression vector, yielding recombinant plasmids for expressing GIPR single-domain antibody-Fc fusion proteins in mammals.

### 5.2. Preparation of Fc fusion proteins of humanized single-domain antibodies

The vectors constructed in 5.1 were transfected into HEK293 cells for transient antibody expression. The recombinant expression plasmids were diluted with Freestyle293 culture medium and added to a PEI (polyethylenimine) solution required for transformation. Each plasmid/PEI mixture was added to a suspension of HEK293 cells, and the cells were cultured at 37 °C with 5% CO2 at 130 rpm. After four hours, EXCELL293 culture medium and 2 mM glutamine were added, and the cells were cultured at 130 rpm. After 24 hours, 3.8 mM VPA was added. After 72 hours, 4 g/L glucose was added. After 5-6 days of culture, the transient expression culture supernatants were collected and purified by Protein A affinity chromatography, yielding the target huGI single-domain antibody-Fc fusion proteins. The purities of the proteins were tested by SDS-PAGE. The expression levels of the proteins are shown in Table 11, and after single-step purification, the SDS purities of the proteins were all greater than 95%.

**Table 11. The expression levels of Fc fusion proteins of the humanized single-domain antibodies**

| Antibody | Expression level (mg/L) |
|---|---|
| huGI-72v1-Ld-Fc | 134 |
| huGI-72v2-Ld-Fc | 119 |
| huGI-72v3-Ld-Fc | 142 |
| huGI-72v4-Ld-Fc | 163 |
| huGI-72v5-Ld-Fc | 220 |
| huGI-72v6-Ld-Fc | 185 |
| huGI-72v7-Ld-Fc | 87 |
| huGI-72v8-Ld-Fc | 318 |
| huGI-1198n1-Ld-Fc | 456 |
| huGI-1198n2-Ld-Fc | 448 |
| huGI-1198n3-Ld-Fc | 528 |
| huGI-1198n4-Ld-Fc | 408 |
| huGI-1198n5-Ld-Fc | 448 |
| huGI-1198n6-Ld-Fc | 469 |
| huGI-1198n7-Ld-Fc | 216 |
| huGI-1198n8-Ld-Fc | 211 |
| huGI-1198n9-Ld-Fc | 204 |
| huGI-1198n10-Ld-Fc | 222 |
| huGI-1198n11-Ld-Fc | 251 |

### Example 6

### Identification of Functions of Fc Fusion Proteins of Humanized Single-Domain Antibodies

### 6.1. Ability of humanized GIPR single-domain antibody-Fc fusion protein to bind to GIPR

293T-GIPR cells were plated in a cell culture plate, and a humanized GIPR single-domain antibody-Fc fusion protein or maridebart sample was added, with final concentrations of 0.00128 nM-100 nM. The anti-human secondary antibody SULFO-TAG (MSD, Cat. No. R32AJ-1) was added, followed by incubation. The mean fluorescence intensity (MFI) was measured using MSD.

Maridebart is an anti-GIPR antibody and was cloned and prepared in-house with reference to the sequence disclosed in WHO Drug Information, Volume 36. Number 4. 2022. Proposed INN: List 128. The construct 2G10 LC1.006 in Patent Application No. WO2017112824 is a similar sequence.

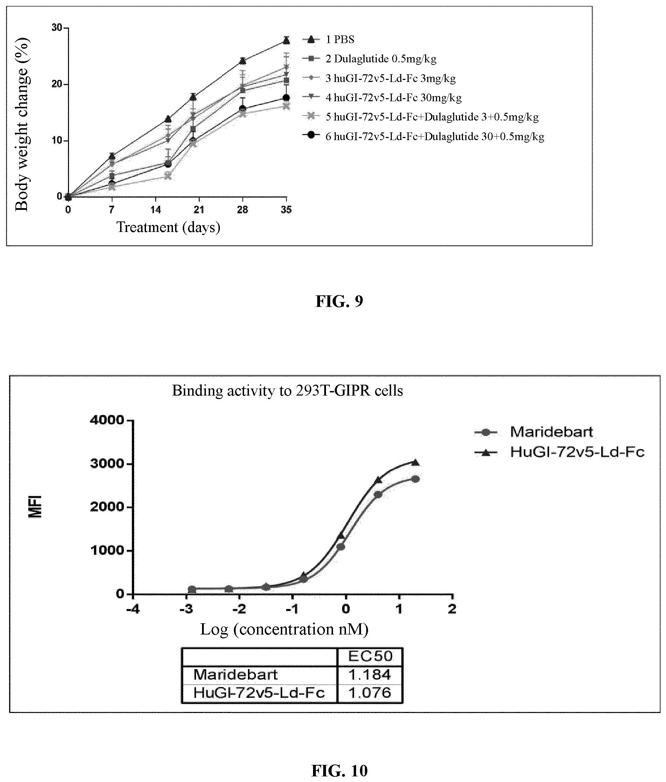

The results are shown in FIG. 10: The ability of the humanized GIPR single-domain antibody-Fc fusion protein to bind to GIPR was slightly superior to that of maridebart.

### 6.2. Affinities of humanized GIPR single-domain antibody-Fc fusion proteins for GIPR

The equilibrium dissociation constants (KD), association rates (Kₐ), and dissociation rates (K_{dis}) of humanized single-domain antibody-Fc fusion proteins to GIPR-ECD-chis were determined and calculated with reference to the experimental steps of 3.3, and the results are shown in Tables 12 to 14: The affinities of the fusion proteins for GIPR after humanization were comparable to those before humanization.

**Table 12. The affinities of humanized GIPR single-domain antibody-Fc fusion proteins for GIPR**

| Antibody | KD (M) | Kₐ (1/Ms) | K_{dis} (1/s) |
|---|---|---|---|
| iGI72-72-Ld-Fc | 1. 19E-09 | 2.39E+05 | 2.85E-04 |
| huGI-72v1-Ld-Fc | 1.17E-09 | 2.23E+05 | 2.60E-04 |
| huGI-72v2-Ld-Fc | 1.74E-09 | 2.12E+05 | 3.70E-04 |
| huGI-72v3-Ld-Fc | 1.56E-09 | 1.64E+05 | 2.56E-04 |
| huGI-72v4-Ld-Fc | 7.16E-10 | 1.66E+05 | 1.19E-04 |
| huGI-72v5-Ld-Fc | 9.03E-10 | 2.02E+05 | 1.82E-04 |
| huGI-72v6-Ld-Fc | 1.23E-09 | 2.30E+05 | 2.83E-04 |
| huGI-72v7-Ld-Fc | 1.53E-09 | 1.80E+05 | 2.76E-04 |

**Table 13. The affinities of humanized GIPR single-domain antibody-Fc fusion proteins for GIPR**

| Antibody | KD (M) | Kₐ (1/Ms) | K_{dis} (1/s) |
|---|---|---|---|
| iGI-1198-Ld-Fc | 1.86E-09 | 8.39E+04 | 1.56E-04 |
| huGI-1198n5-Ld-Fc | 2.09E-09 | 8.10E+04 | 1.69E-04 |
| huGI-1198n6-Ld-Fc | 2.83E-09 | 8.55E+04 | 2.42E-04 |
| huGI-1198n4-Ld-Fc | 2.32E-09 | 8.81E+04 | 2.05E-04 |

**Table 14. The affinities of humanized GIPR single-domain antibody-Fc fusion proteins for GIPR**

| Antibody | KD (M) | Kₐ (1/Ms) | K_{dis} (1/s) |
|---|---|---|---|
| iGI-1198-Ld-Fc | 3.502E-09 | 6.341E+04 | 2.221E-04 |
| huGI-1198n7-Ld-Fc | 2.458E-09 | 8.135E+04 | 2.000E-04 |
| huGI-1198n8-Ld-Fc | 2.940E-09 | 7.417E+04 | 2.181E-04 |
| huGI-1198n9-Ld-Fc | 3.703E-09 | 6.804E+04 | 2.519E-04 |
| huGI-1198n10-Ld-Fc | 5.417E-09 | 6.629E+04 | 3.591E-04 |
| huGI-1198n11-Ld-Fc | 5.098E-09 | 7.443E+04 | 3.794E-04 |

The equilibrium dissociation constant (KD), association rate (Kₐ), and dissociation rate (K_{dis}) of a humanized single-domain antibody-Fc fusion protein to GIPR-ECD-chis were determined by the same experimental steps and compared with those of maridebart. The results are shown in Table 15: The affinity of the humanized single-domain antibody-Fc fusion protein was superior to that of the antibody maridebart.

**Table 15. The affinity of a humanized GIPR single-domain antibody-Fc fusion protein for GIPR**

| Antibody | KD (M) | Kₐ (1/Ms) | K_{dis} (1/s) |
|---|---|---|---|
| Maridebart | 1.627E-08 | 1.364E+05 | 2.220E-03 |
| huGI-72v5-Ld-Fc | 2.766E-09 | 2.078E+05 | 5.747E-04 |

### 6.3. Neutralizing activity of humanized GIPR single-domain antibody-Fc fusion proteins

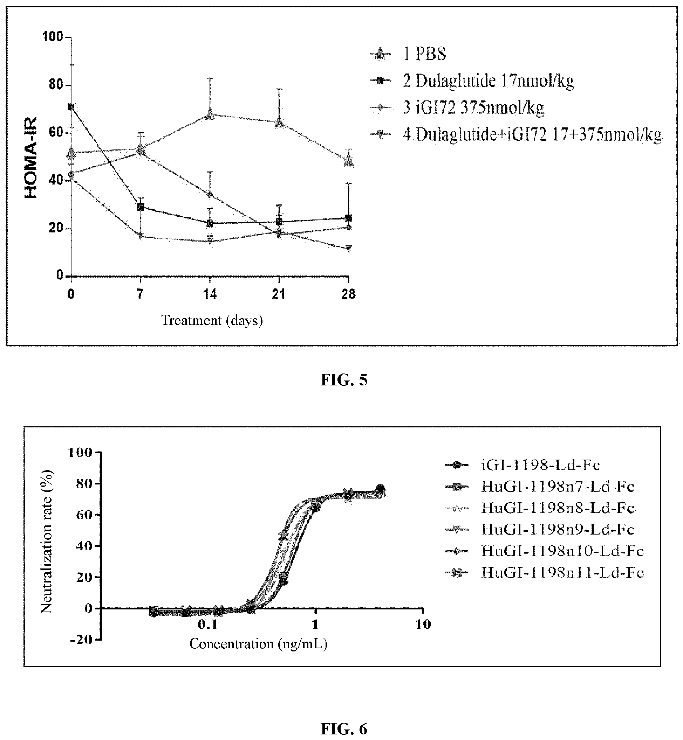

The neutralizing activity of humanized GIPR single-domain antibody-Fc fusion proteins was determined with reference to the experimental steps of Example 3.2, and the results are shown in FIG. 6: The humanized GIPR single-domain antibody-Fc fusion proteins were all superior in neutralizing activity to the non-humanized molecule, the iGI-1198-Fc fusion protein.

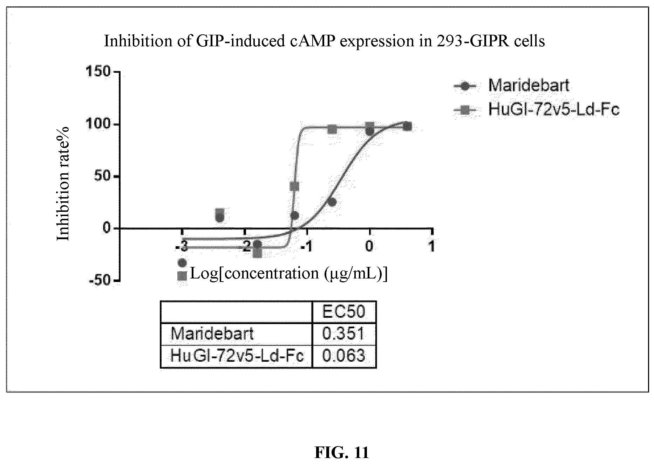

GIP-C12H4-Fc4 and a humanized GIPR single-domain antibody-Fc or maridebart sample (25 µL) were separately added to a 96-well plate, and 293T-GIPR cells were then added. After incubation, an assay was performed using a cAMP kit. The absorbance at 450 nm/550 nm was measured using a microplate reader, and the inhibition rate (%) was calculated. The results are shown in FIG. 11: The humanized GIPR single-domain antibody-Fc fusion protein was superior in neutralizing activity to maridebart.

### 6.4. Study of in vivo efficacy of humanized GIPR single-domain antibody-Fc fusion protein

### (1) Experiment of stimulating C57BL/6 mice with DA-GIP

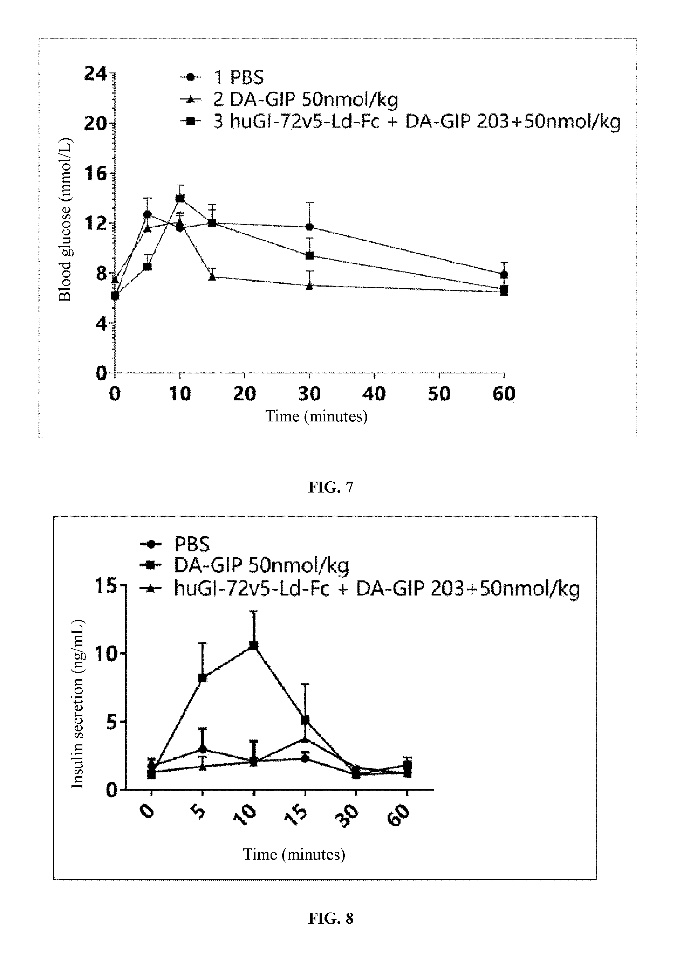

After nine C57BL/6 mice were fasted overnight, their blood glucose levels were measured, and they were randomized into 3 groups. Twenty-four hours after returning to a normal diet, huGI-72v5-Ld-Fc 203 nmol/kg was administered according to the experimental protocol. Twelve hours after the administration, mice were fasted overnight. Forty-eight hours after the administration, each mouse was intraperitoneally given DA-GIP 50 nmol/kg. Immediately after the administration of DA-GIP, a 2 g/kg oral glucose load was administered. Blood samples were taken before the glucose load and 5, 10, 15, 30, and 60 min after the load and assayed for blood glucose and insulin levels. The results are shown in FIGs. 7-8: Compared to the PBS group, DA-GIP significantly reduced OGTT blood glucose. HuGI-72v5-Ld-Fc blocked the hypoglycemic effect of DA-GIP, indicating that huGI-72v5-Ld-Fc has significant blocking activity in mice.

### (2) Multiple administrations to ob mice

Twenty-four ob/ob mice were randomized into 6 groups according to blood glucose and body weight, and administration was started at 6-8 weeks. The day of grouping and administration is denoted by D0. Administration was performed once a week and 6 times in total. Body weight was measured before administration and on D0, 7, 16, 20, 28, and 35 post-administration. The results are shown in FIG. 9: The dulaglutide 0.5 mg/kg, huGI-72v5-Ld-Fc 3 mg/kg, and huGI-72v5-Ld-Fc 30 mg/kg treatment groups all exhibited significant reductions in the body weight of ob/ob mice, and the dulaglutide + huGI-72v5-Ld-Fc treatment group exhibited a further enhanced body weight-reducing effect.

The foregoing detailed description is provided by way of illustration and example and is not intended to limit the scope of the appended claims. Various variants of the embodiments currently listed in the present disclosure will be apparent to those of ordinary skill in the art and are intended to be within the scope of the appended claims and equivalents thereof.

### Sequence information

iGI-9 (SEQ ID NO: 1):

QVQLVESGGGLVQPGGSLRLSCETSKILFSSAYTMGWYRQAPGKQRELVATITTDYITNYVD
SVKGRFTISRDNAKNTVYLQMNTLKLEDTAVYYCNARWGRDYWGQGTQVTVSS

| | Kabat | AbM | Chothia | IMGT |
|---|---|---|---|---|
| CDR1 | SAYTMG (SEQ ID NO: 26) | KILFSSAYTMG (SEQ ID NO: 29) | KILFSSAY (SEQ ID NO: 32) | KILFSSAYT (SEQ ID NO: 35) |
| CDR2 | TITTDYITNYVDS VKG (SEQ ID NO: 27) | TITTDYITN (SEQ ID NO: 30) | TTDYI (SEQ ID NO: 33) | ITTDYIT (SEQ ID NO: 36) |
| CDR3 | RWGRDY (SEQ ID NO: 28) | RWGRDY (SEQ ID NO: 31) | RWGRDY (SEQ ID NO: 34) | NARWGRDY (SEQ ID NO: 37) |

iGI-27 (SEQ ID NO: 2):

QVQLVESGGALVQTGGSLRLFCAASGDTICITNMNWYRQAPGKGREFVAAITRSGRTLYAD
YVKGRFTISRDNARNTMSLQMNSMTSEDTAVYYCNADQNQTICAAEPSAWGRGTQVTVSS

| | Kabat | AbM | Chothia | IMGT |
|---|---|---|---|---|
| CDR1 | ITNMN (SEQ ID NO: 38) | GDTICITNMN (SEQ ID NO: 41) | GDTICIT (SEQ ID NO: 44) | GDTICITN (SEQ ID NO: 47) |
| CDR2 | AITRSGRTLYADYV KG (SEQ ID NO: 39) | AITRSGRTL (SEQ ID NO: 42) | TRSGR (SEQ ID NO: 45) | ITRSGRT (SEQ ID NO: 48) |
| CDR3 | DQNQTICAAEPSA (SEQ ID NO: 40) | DQNQTICAAEP SA (SEQ ID NO: 43) | DQNQTICAAEP SA (SEQ ID NO: 46) | NADQNQTICAAEP SA (SEQ ID NO: 49) |

iGI-44 (SEQ ID NO: 3):

QVQLVESGGGLVQAGGSLKLSCAASGRTFSSKAMGWFRQAPGKEREFVAAINWSGDRTYH
VNSIKGRFTISRDNAKNTVYLQMNSLKPEDTAVYYCTADRRDRATIPFQWHYWGQGTQVT
VSS

| | Kabat | AbM | Chothia | IMGT |
|---|---|---|---|---|
| CDR1 | SKAMG (SEQ ID NO: 50) | GRTFSSKAMG (SEQ ID NO: 53) | GRTFSSK (SEQ ID NO: 56) | GRTFSSKA (SEQ ID NO: 59) |
| CDR2 | AINWSGDRTY HVNSIKG (SEQ ID NO: 51) | AINWSGDRTY (SEQ ID NO: 54) | NWSGDR (SEQ ID NO: 57) | INWSGDRT (SEQ ID NO: 60) |
| CDR3 | DRRDRATIPFQ WHY (SEQ ID NO: 52) | DRRDRATIPFQ WHY (SEQ ID NO: 55) | DRRDRATIPFQ WHY (SEQ ID NO: 58) | TADRRDRATIP FQWHY (SEQ ID NO: 61) |

iGI-51 (SEQ ID NO: 4):

QVQLVESGGKLVQAGGSLRLDCVASGRTFSYYAIGWYRQAPGKEREFVAAIRASGGSTYYA
DSVKGRFTASRDNAKNTGYLQMNSLKPEDTAVYFCYAATIVPITPSTHGYWGQGTQVTVSS

| | Kabat | AbM | Chothia | IMGT |
|---|---|---|---|---|
| CDR1 | YYAIG (SEQ ID NO: 62) | GRTFSYYAIG (SEQ ID NO: 65) | GRTFSYY (SEQ ID NO: 68) | GRTFSYYA (SEQ ID NO: 71) |
| CDR2 | AIRASGGSTYY ADSVKG (SEQ ID NO: 63) | AIRASGGSTY (SEQ ID NO: 66) | RASGGS (SEQ ID NO: 69) | IRASGGST (SEQ ID NO: 72) |
| CDR3 | ATIVPITPSTHG Y (SEQ ID NO: 64) | ATIVPITPSTHG Y (SEQ ID NO: 67) | ATIVPITPSTHG Y (SEQ ID NO: 70) | YAATIVPITPST HGY (SEQ ID NO: 73) |

iGI-72 (SEQ ID NO: 5):

QVQLVESGGGLVQPGGSLRLSCAASRYTLDYYAIGWFRQAPGKEREGVSCINSKDGSTYYA
DSVKGRFTISKDNAKNTVYLQMNSLKPEDTAVYYCAAGYDPWSGIPPVQAMCVMGYDY
WGQGTQVTVSS

| | Kabat | AbM | Chothia | IMGT |
|---|---|---|---|---|
| CDR1 | YYAIG (SEQ ID NO: 74) | RYTLDYYAIG (SEQ ID NO: 77) | RYTLDYY (SEQ ID NO: 80) | RYTLDYYA (SEQ ID NO: 83) |
| CDR2 | CINSKDGSTYYA DSVKG (SEQ ID NO: 75) | CINSKDGSTY (SEQ ID NO: 78) | NSKDGS (SEQ ID NO: 81) | INSKDGST (SEQ ID NO: 84) |
| CDR3 | GYDPWSGIPPVQ AMCVMGYDY (SEQ ID NO: 76) | GYDPWSGIPPVQ AMCVMGYDY (SEQ ID NO: 79) | GYDPWSGIPPVQ AMCVMGYDY (SEQ ID NO: 82) | AAGYDPWSGIPP VQAMCVMGYD Y (SEQ ID NO: 85) |

iGI-1198 (SEQ ID NO: 6):

QVQLQESGGGLVQPGGSLRLSCVASGSGFSIVAMGWYRQTPGKQRELVAAITSGGNTNYAD
SVKGRFTISRDNAKNTIYLQMNSLKPEDTAVYYCNADPGVVAGPYAGMDFWGKGTQVTVS
S

| | Kabat | AbM | Chothia | IMGT |
|---|---|---|---|---|
| CDR1 | IVAMG (SEQ ID NO: 86) | GSGFSIVAMG (SEQ ID NO: 89) | GSGFSIV (SEQ ID NO: 92) | GSGFSIVA (SEQ ID NO: 95) |
| CDR2 | AITSGGNTNYA DSVKG (SEQ ID NO: 87) | AITSGGNTN (SEQ ID NO: 90) | TSGGN (SEQ ID NO: 93) | ITSGGNT (SEQ ID NO: 96) |
| CDR3 | DPGVVAGPYA GMDF (SEQ ID NO: 88) | DPGVVAGPYAG MDF (SEQ ID NO: 91) | DPGVVAGPYAG MDF (SEQ ID NO: 94) | NADPGVVAGPYAG MDF (SEQ ID NO: 97) |

iGI-1225NA (SEQ ID NO: 7):

QVQLQESGGSLRLSCAASGPIFSFTTMAWYRQVPGKQRELVASITTGGSTAYTDSVKGRFTI
SRDNAKSTLYLQMNNLKPEDTAVYYCNTGPRNVWAAAWGQGTQVTVSS

| | Kabat | AbM | Chothia | IMGT |
|---|---|---|---|---|
| CDR1 | FTTMA (SEQ ID NO: 98) | GPIFSFTTMA (SEQ ID NO: 101) | GPIFSFT (SEQ ID NO: 104) | GPIFSFTT (SEQ ID NO: 107) |
| CDR2 | SITTGGSTAYTDSV KG (SEQ ID NO: 99) | SITTGGSTA (SEQ ID NO: 102) | TTGGS (SEQ ID NO: 105) | ITTGGST (SEQ ID NO: 108) |
| CDR3 | GPRNVWAAA (SEQ ID NO: 100) | GPRNVWAAA (SEQ ID NO: 103) | GPRNVWAAA (SEQ ID NO: 106) | NTGPRNVWAAA (SEQ ID NO: 109) |

huGI-72v1 (SEQ ID NO: 8): 
EVQLVESGGGLVQPGGSLRLSCAASRYTLDYYAIGWFRQAPGKEREGVSCINSKDGSTYYA
DSVKGRFTISKDNSKNTVYLQMNSLRAEDTAVYYCAAGYDPWSGIPPVQAMCVMGYDY
WGQGTLVTVSS
huGI-72v2 (SEQ ID NO: 9): 
EVQLVESGGGLVQPGGSLRLSCAASRYTLDYYAIGWFRQAPGKEREGVSCINSKDGSTYYA
DSVKGRFTISRDNSKNTVYLQMNSLRAEDTAVYYCAAGYDPWSGIPPVQAMCVMGYDYW
GQGTLVTVSS
huGI-72v3 (SEQ ID NO: 10): 
EVQLVESGGGLVQPGGSLRLSCAASRYTLDYYAIGWFRQAPGKGLEGVSCINSKDGSTYYA
DSVKGRFTISKDNSKNTVYLQMNSLRAEDTAVYYCAAGYDPWSGIPPVQAMCVMGYDY
WGQGTLVTVSS
huGI-72v4 (SEQ ID NO: 11): EVQLVESGGGLVQPGGSLRLSCAASRYTLDYYAIGWFRQAPGKGLEGVSCINSKDGSTYYA 
DSVKGRFTISRDNSKNTVYLQMNSLRAEDTAVYYCAAGYDPWSGIPPVQAMCVMGYDYW

GQGTLVTVSS
huGI-72v5 (SEQ ID NO: 12): 
QVQLVESGGGLVQPGGSLRLSCSASRYTLDYYAIGWFRQAPGKEREGVSCINSKDGSTYYA
DSVKGRFTISKDNSKNTVYLQMNSLRAEDTAVYYCAAGYDPWSGIPPVQAMCVMGYDY
WGQGTLVTVSS
huGI-72v6 (SEQ ID NO: 13): 
QVQLVESGGGLVQPGGSLRLSCSASRYTLDYYAIGWFRQAPGKEREGVSCINSKDGSTYYA
DSVKGRFTISRDNSKNTVYLQMNSLRAEDTAVYYCAAGYDPWSGIPPVQAMCVMGYDYW
GQGTLVTVSS
huGI-72v7 (SEQ ID NO: 14): 
QVQLVESGGGLVQPGGSLRLSCSASRYTLDYYAIGWFRQAPGKGLEGVSCINSKDGSTYYA
DSVKGRFTISKDNSKNTVYLQMNSLRAEDTAVYYCAAGYDPWSGIPPVQAMCVMGYDY
WGQGTLVTVSS
huGI-1198n1 (SEQ ID NO: 15): 
QVQLVESGGGLVQPGGSLRLSCSASGSGFSIVAMGWVRQAPGKGLELVAAITSGGNTNYAD
SVKGRFTISRDNSKNTIYLQMNSLRAEDTAVYYCNADPGVVAGPYAGMDFWGKGTTVTVS
S
huGI-1198n2 (SEQ ID NO: 16): 
QVQLQESGGGLVQPGGSLRLSCSASGSGFSIVAMGWVRQTPGKGLELVAAITSGGNTNYAD
SVKGRFTISRDNSKNTIYLQMNSLRAEDTAVYYCNADPGVVAGPYAGMDFWGKGTTVTVS
S
huGI-1198n3 (SEQ ID NO: 17): 
QVQLQESGGGLVQPGGSLRLSCSASGSGFSIVAMGWVRQTPGKGLELVAAITSGGNTNYAD
SVKGRFTISRDNAKNTIYLQMNSLRAEDTAVYYCNADPGVVAGPYAGMDFWGKGTTVTVS
S
huGI-1198n4 (SEQ ID NO: 18): 
QVQLVESGGGLVQPGGSLRLSCSASGSGFSIVAMGWVRQAPGKQRELVAAITSGGNTNYAD
SVKGRFTISRDNSKNTIYLQMNSLRAEDTAVYYCNADPGVVAGPYAGMDFWGKGTTVTVS
S
huGI-1198n5 (SEQ ID NO: 19): 
QVQLQESGGGLVQPGGSLRLSCSASGSGFSIVAMGWVRQTPGKQRELVAAITSGGNTNYAD
SVKGRFTISRDNAKNTIYLQMNSLRAEDTAVYYCNADPGVVAGPYAGMDFWGKGTTVTVS
S
huGI-1198n6 (SEQ ID NO: 20): 
QVQLQESGGGLVQPGGSLRLSCSASGSGFSIVAMGWVRQTPGKQRELVAAITSGGNTNYAD

SVKGRFTISRDNAKNTIYLQMNSLKPEDTAVYYCNADPGVVAGPYAGMDFWGKGTTVTVS

S
huGI-1198n7 (SEQ ID NO: 21): 
QVQLVESGGGLVQPGGSLRLSCSASGSGFSIVAMGWYRQTPGKGLELVAAITSGGNTNYAD
SVKGRFTISRDNSKNTIYLQMNSLRAEDTAVYYCNADPGVVAGPYAGMDFWGKGTTVTVS
S
huGI-1198n8 (SEQ ID NO: 22): 
QVQLQESGGGLVQPGGSLRLSCSASGSGFSIVAMGWYRQTPGKGLELVAAITSGGNTNYAD
SVKGRFTISRDNSKNTIYLQMNSLRAEDTAVYYCNADPGVVAGPYAGMDFWGKGTTVTVS
S
huGI-1198n9 (SEQ ID NO: 23): 
QVQLVESGGGLVQPGGSLRLSCSASGSGFSIVAMGWYRQTPGKQRELVAAITSGGNTNYAD
SVKGRFTISRDNSKNTIYLQMNSLRAEDTAVYYCNADPGVVAGPYAGMDFWGKGTTVTVS
S
huGI-1198n10 (SEQ ID NO: 24): 
QVQLQESGGGLVQPGGSLRLSCSASGSGFSIVAMGWYRQTPGKQRELVAAITSGGNTNYAD
SVKGRFTISRDNAKNTIYLQMNSLRAEDTAVYYCNADPGVVAGPYAGMDFWGKGTTVTVS
S
huGI-1198n11 (SEQ ID NO: 25): 
QVQLQESGGGLVQPGGSLRLSCSASGSGFSIVAMGWYRQTPGKQRELVAAITSGGNTNYAD
SVKGRFTISRDNAKNTIYLQMNSLKPEDTAVYYCNADPGVVAGPYAGMDFWGKGTTVTVS
S
IgG1 Fc (SEQ ID NO: 110) 
EPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK
IgG1 Fc (C220S) (SEQ ID NO: 113)

EPKSSDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK

## Claims

1. A glucose-dependent insulinotropic polypeptide receptor (GIPR)-binding protein, comprising at least one immunoglobulin single variable domain, wherein the at least one immunoglobulin single variable domain comprises a CDR1, a CDR2, and a CDR3 from a VHH set forth in SEQ ID NO: 5 and/or SEQ ID NO: 6.

2. The GIPR-binding protein according to claim 1, wherein the CDR1, CDR2, and CDR3 are defined according to the following definition system: Kabat, AbM, Chothia, or IMGT.

3. The GIPR-binding protein according to claim 1 or 2, wherein the immunoglobulin single variable domain is from the family Camelidae, humanized, or chimeric.

4. The GIPR-binding protein according to any one of claims 1-3, wherein the CDR1, CDR2, and CDR3 from the VHH set forth in SEQ ID NO: 5 are selected from any one of the following groups: SEQ ID NOs: 74-76, SEQ ID NOs: 77-79, SEQ ID NOs: 80-82, and SEQ ID NOs: 83-85.

5. The GIPR-binding protein according to claim 4, wherein the at least one immunoglobulin single variable domain comprises one or more of the amino acid sequences set forth in SEQ ID NO: 5 and SEQ ID NOs: 8-14.

6. The GIPR-binding protein according to claim 5, wherein the at least one immunoglobulin single variable domain comprises the amino acid sequence set forth in one of SEQ ID NO: 5 and SEQ ID NOs: 8-14.

7. The GIPR-binding protein according to any one of claims 1-3, wherein the CDR1, CDR2, and CDR3 from the VHH set forth in SEQ ID NO: 6 are selected from any one of the following groups: SEQ ID NOs: 86-88, SEQ ID NOs: 89-91, SEQ ID NOs: 92-94, and SEQ ID NOs: 95-97.

8. The GIPR-binding protein according to claim 7, wherein the at least one immunoglobulin single variable domain comprises one or more of the amino acid sequences set forth in SEQ ID NO: 6 and SEQ ID NOs: 15-25.

9. The GIPR-binding protein according to claim 8, wherein the at least one immunoglobulin single variable domain comprises the amino acid sequence set forth in one of SEQ ID NO: 6 and SEQ ID NOs: 15-25.

10. The GIPR-binding protein according to any one of claims 1-9, wherein the at least one immunoglobulin single variable domain comprises one or more of the amino acid sequences set forth in SEQ ID NO: 5 and SEQ ID NOs: 8-14, and one or more of the amino acid sequences set forth in SEQ ID NO: 6 and SEQ ID NOs: 15-25.

11. The GIPR-binding protein according to claim 10, wherein the at least one immunoglobulin single variable domain comprises the amino acid sequence set forth in one of SEQ ID NO: 5 and SEQ ID NOs: 8-14 and the amino acid sequence set forth in one of SEQ ID NO: 6 and SEQ ID NOs: 15-25.

12. The GIPR-binding protein according to any one of claims 1-11, wherein the GIPR-binding protein comprises one said immunoglobulin single variable domain.

13. The GIPR-binding protein according to any one of claims 1-11, wherein the GIPR-binding protein comprises multiple, for example, 2, 3, 4, or 5, said immunoglobulin single variable domains.

14. The GIPR-binding protein according to any one of claims 1-13, further comprising an immunoglobulin Fc region, preferably a human immunoglobulin Fc region, and more preferably a human IgG1, IgG2, IgG3, or IgG4 Fc region.

15. The GIPR-binding protein according to claim 14, wherein the amino acid sequence of the immunoglobulin Fc region is set forth in SEQ ID NO: 110 or SEQ ID NO: 113.

16. The GIPR-binding protein according to claim 14 or 15, wherein the immunoglobulin Fc region is linked directly or linked indirectly by a linker to the at least one immunoglobulin single variable domain.

17. The GIPR-binding protein according to any one of claims 1-16, having at least one of the following characteristics:
(a) having specific binding to human GIPR;
(b) binding to human GIPR with a KD value of less than 1 × 10⁻⁷ M, preferably less than 1 × 10⁻⁸ M;
(c) blocking the interaction of GIP with GIPR; and
(d) being capable of reducing the body weight and/or blood glucose of a subject.

18. A fusion protein, comprising the GIPR-binding protein according to any one of claims 1-17.

19. A multispecific antibody, comprising the GIPR-binding protein according to any one of claims 1-17 and/or the fusion protein according to claim 18, and one or more additional antigen-binding regions that, compared to the GIPR-binding protein and/or the fusion protein, bind to a different antigen or a different epitope of the same antigen.

20. A conjugate, comprising the GIPR-binding protein according to any one of claims 1-17 and/or the fusion protein according to claim 18 and/or the multispecific antibody according to claim 19, wherein the conjugate further comprises a molecule conjugated to the GIPR-binding protein and/or the fusion protein and/or the multispecific antibody.

21. A nucleic acid molecule, wherein the nucleic acid molecule encodes the GIPR-binding protein according to any one of claims 1-17 and/or the fusion protein according to claim 18 and/or the multispecific antibody according to claim 19.

22. An expression vector, comprising the nucleic acid molecule according to claim 20 operably linked to an expression control element.

23. A recombinant cell, comprising the nucleic acid molecule according to claim 21 and/or transformed with the expression vector according to claim 22 and capable of expressing the GIPR-binding protein and/or the fusion protein and/or the multispecific antibody.

24. A pharmaceutical composition, comprising the GIPR-binding protein according to any one of claims 1-17 and/or the fusion protein according to claim 18 and/or the multispecific antibody according to claim 19 and/or the conjugate according to claim 20 and/or the nucleic acid molecule according to claim 21 and/or the expression vector according to claim 22 and/or the recombinant cell according to claim 23 and a pharmaceutically acceptable carrier.

25. Akit, comprising the GIPR-binding protein according to any one of claims 1-17, the fusion protein according to claim 18, the multispecific antibody according to claim 19, the conjugate according to claim 20, and/or the pharmaceutical composition according to claim 24.

26. A method for determining the presence and/or content of a GIPR protein, comprising providing the GIPR-binding protein according to any one of claims 1-17, the fusion protein according to claim 18, the multispecific antibody according to claim 19, and/or the conjugate according to claim 20.

27. A method for inhibiting binding of a GIPR protein to a ligand thereof, comprising providing the GIPR-binding protein according to any one of claims 1-17, the fusion protein according to claim 18, the multispecific antibody according to claim 19, and/or the conjugate according to claim 20, wherein preferably, the ligand is GIP.

28. A method for treating and/or preventing a metabolic disease and/or symptom, comprising administering to a subject in need thereof an effective amount of the GIPR-binding protein according to any one of claims 1-17, the fusion protein according to claim 18, the multispecific antibody according to claim 19, the conjugate according to claim 20, and/or the pharmaceutical composition according to claim 24.

29. The method according to claim 28, wherein the metabolic disease and/or symptom is obesity, overweight, and/or diabetes.
